(19) Europäisches Patentamt / European Patent Office / Office européen des brevets

(11) **EP 3 566 769 B1**

(12) **FASCICULE DE BREVET EUROPEEN**

(45) Date de publication et mention
de la délivrance du brevet:
**26.07.2023   Bulletin 2023/30**

(21) Numéro de dépôt: **18171093.0**

(22) Date de dépôt: **07.05.2018**

(51) Classification Internationale des Brevets (IPC):
**B01J 19/00** [(2006.01)]    **G16C 10/00** [(2019.01)]

(52) Classification Coopérative des Brevets (CPC):
**B01J 19/0006; B01J 19/0033;** B01J 2219/00243;
G16C 20/10

(54) **METHODE ET DISPOSITIF DE SIMULATION D'UNE INSTALLATION INDUSTRIELLE POUR L'EXPLOITATION D'UN PROCEDE CHIMIQUE OU BIOCHIMIQUE**

SIMULATIONSMETHODE UND -VORRICHTUNG EINER INDUSTRIEANLAGE FÜR DIE NUTZUNG EINES CHEMISCHEN ODER BIOCHEMISCHEN VERFAHRENS

METHOD AND DEVICE FOR SIMULATING AN INDUSTRIAL FACILITY FOR EXPLOITING A CHEMICAL OR BIOCHEMICAL METHOD

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(43) Date de publication de la demande:
**13.11.2019   Bulletin 2019/46**

(73) Titulaire: **YF1**
**54000 Nancy (FR)**

(72) Inventeurs:
• **NICOUD, Roger-Marc**
**54690 Lay-St-Christophe (FR)**

• **BERGER, Etienne**
**54000 Nancy (FR)**
• **SIMON, Marc-Olivier**
**69100 Villeurbanne (FR)**

(74) Mandataire: **August Debouzy**
**7, rue de Téhéran**
**75008 Paris (FR)**

(56) Documents cités:
**EP-A1- 3 242 226      WO-A1-2004/045760**
**WO-A1-2004/081680      US-A1- 2002 143 725**
**US-A1- 2005 187 745      US-B1- 9 183 347**

## Description

### Domaine technique

L'invention se rapporte à une méthode de simulation d'une installation industrielle pour l'exploitation d'un procédé d'obtention de molécules chimiques ou de matière biologique. L'invention porte également sur un dispositif pour la mise en oeuvre au moyen d'un ordinateur de cette méthode, sur un programme d'ordinateur pour l'exécution de la méthode, et sur une installation industrielle obtenue par ladite méthode.

### Etat de la technique

Les industriels de la chimie fine ou des biotechnologies doivent fréquemment estimer ou comparer des procédés de production chimiques ou biochimiques avec une quantité d'information très limitée, tout particulièrement dans les premiers stades de développement. Notons que dans ces industries, lorsqu'un procédé de production doit être imaginé, il arrive fréquemment que les chances de mise sur le marché de la molécule visée soient très faibles, par exemple, pour des raisons d'échec d'essais cliniques. Il existe donc de très nombreuses estimations technico-économiques à effectuer sur de nombreuses molécules qui ne seront produites qu'en faible quantités et pendant une période de temps limité. La capacité à disposer d'estimations rapides est donc primordiale.

A l'heure actuelle, les ingénieurs et managers gèrent ce problème par des approches très expérimentales ou basées sur une certaine expérience du domaine, les estimations très approximatives étant effectuées avec des outils de type Microsoft Excel®.

Les méthodes éprouvées du génie des procédés, basées sur des modélisations détaillées effectuées avec des outils de simulations commerciaux tels que Honeywell UNISIM®, Aspen HYSYS® ou autres ne sont pratiquement pas utilisées, contrairement à ce qui se passe par exemple en pétrochimie ou en chimie de commodités.

La raison est que ces approches basées sur la simulation nécessitent la connaissance d'un très grand nombre de paramètres physico-chimiques en particulier thermodynamiques, qui sont de fait inconnus et dont la détermination serait incompatible en temps et en coût avec les contraintes de développement de procédés de la chimie fine ou des biotechnologies.

Le peu d'utilisation de ces outils de simulation entraine une relative sous-représentation de personnel spécialisé en génie des procédés, ce qui rend l'interaction avec les chimistes et biochimistes à l'origine du développement encore plus difficile.

La prise de conscience de la difficulté à utiliser des outils de simulation performants lorsque l'on ne dispose que d'un nombre limité d'informations n'est pas nouvelle. En particulier, le document US 8,983,815 divulgue un appareil et une méthode de modélisation informatique de procédés chimiques dans lequel on fournit à un système de simulation un ensemble de données permettant d'obtenir une simulation rigoureuse du procédé. Les exemples fournis dans ce document concernent une installation de séparation de pétrole et de gaz, et une installation de raffinage de pétrole et incluant des considérations technico-économiques qui ne sont pas adaptées à la chimie fine ou aux biotechnologies. La méthode présentée dans le document US 8,983,815 n'est donc pas adaptée à la chimie fine ou aux biotechnologies.

Par « chimie fine » également appelée « chimie de spécialité » on entend dans le présent document une branche de l'industrie chimique qui synthétise des produits spécifiques, dans de faibles volumes de production, mais à haute valeur ajoutée, et répondant à des contraintes techniques, par exemple de pureté, élevées. Par matière biologique, on entend toutes molécules ou mélanges de molécules obtenus par fermentation, culture de cellules ou extraits du milieu naturel. EP-A-3 242 226 décrit une méthode de simulation d'un procédé chimique.

### Résumé de l'invention

Les modes de réalisation de l'invention offrent un outil industriel d'ingénierie des procédés de chimie fine. Une caractéristique technique fonctionnelle des modes de réalisation réside dans la simulation de procédés pour leur mise en oeuvre ultérieure dans des installations de production de produits chimiques ou biologiques.

Les modes de réalisation permettent de prédire, de façon concrète le comportement de procédés chimiques pour faire un certain nombre d'estimations, notamment sur leur faisabilité industrielle. Les modes de réalisation de l'invention permettent ainsi d'orienter le développement de procédés chimiques avec une précision suffisante, et dans des temps et des coûts raisonnables, pour permettre d'estimer les chances de réussite de leur mise en oeuvre industrielle et ce, avant même la mise en place d'une installation et la mise en fonctionnement de cette installation.

Une mise en oeuvre intellectuelle d'une simulation de procédés chimiques n'est pas possible pour des besoins industriels. Il est pratiquement impossible de réaliser les calculs nécessaires pour arriver à des résultats suffisamment précis et significatifs pour prendre des décisions industrielles de mise en oeuvre du procédé ou encore la mise en place d'installations chimique pour une telle mise en oeuvre. La raison en est le temps prohibitif qu'il faudrait passer à caler les paramètres du modèle.

Or, sans une simulation assistée par ordinateur, il est impossible de tester de façon prédictive ou de faire un choix parmi une pluralité de projets de procédés chimiques ceux qui offrent les meilleurs performances, et ce, dans un temps industriellement raisonnable.

Il n'existe aucune méthode purement intellectuelle, mathématique ou encore théorique qui permette de prédire de manière exhaustive et rapide le comportement d'un procédé de chimie fine.

Un but de l'invention est de permettre aux industries de la chimie fine et des biotechnologies l'utilisation de simulateurs pertinents en fournissant un outil utilisable par un non expert de la simulation, qui autorise la prise en compte d'un projet de production avec des connaissances de physico-chimie initialement quasi inexistantes.

Cet outil doit avoir la capacité d'aider l'utilisateur à identifier les points délicats ou limitants du procédé en évaluation afin de déterminer les connaissances à acquérir ou à utiliser afin d'atteindre un objectif donné, à affiner ces prévisions au fur à et mesure de la disposition des nouvelles connaissances. Cet outil devra de plus être fiable, facile à utiliser, et permettre une précision d'estimation des performances technico-économiques du procédé étudié qui soit adaptée au problème posé et qui pourra évoluer en fonction des besoins et de l'évolution du projet industriel

L'idée est de permettre à l'utilisateur de répondre à des questions aussi diverses que :

- Quel serait le coût de production de la molécule visée avec le procédé étudié ?
- Quelle est l'énergie, quelles sont les quantités de matières nécessaires ? De façon plus générale, quel est l'impact environnemental ?
- Quelles sont les étapes sur lesquelles l'effort doit se concentrer à des fins d'amélioration ?
- Est-il préférable d'opérer le procédé en discontinu ou en continu ?
- Quels sont les leviers pour améliorer une pureté ? Un rendement ?

L'invention est définie par les revendications indépendantes. Les revendications dépendantes définissent des modes de réalisations préférés.

## Brève description des dessins

Ces aspects et d'autres aspects de l'invention seront expliqués plus en détail au moyen du/des modes de réalisation de l'invention décrits ci-après à titre d'exemple(s), en référence aux dessins annexés, sur lesquels

la Fig. 1 est un diagramme d'opération du procédé traditionnel de purification de la DVL,
la Fig. 2 est un diagramme d'opérations du procédé de purification de la DVL avec recyclage du solvant d'extraction,
la Fig. 3 est un diagramme d'opérations du procédé de purification de la DVL par neutralisation thermique du peroxyde,

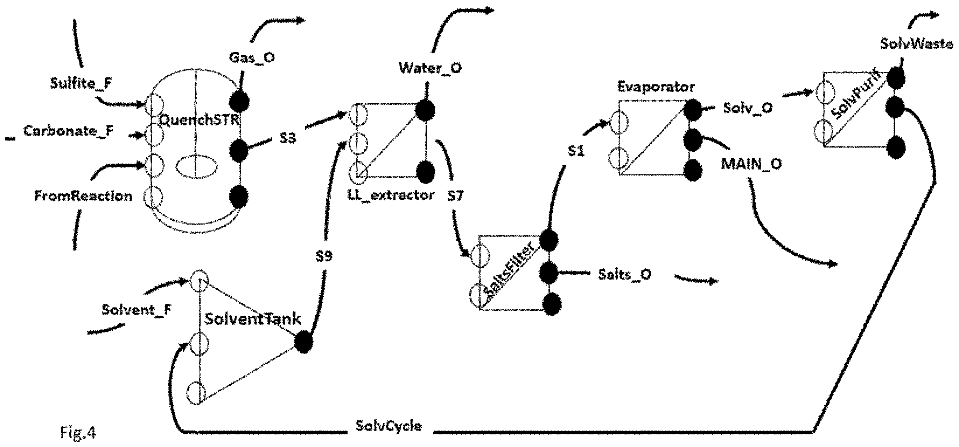

la Fig. 4 est un diagramme d'équipements du procédé de purification de la DVL avec recyclage du solvant d'extraction,

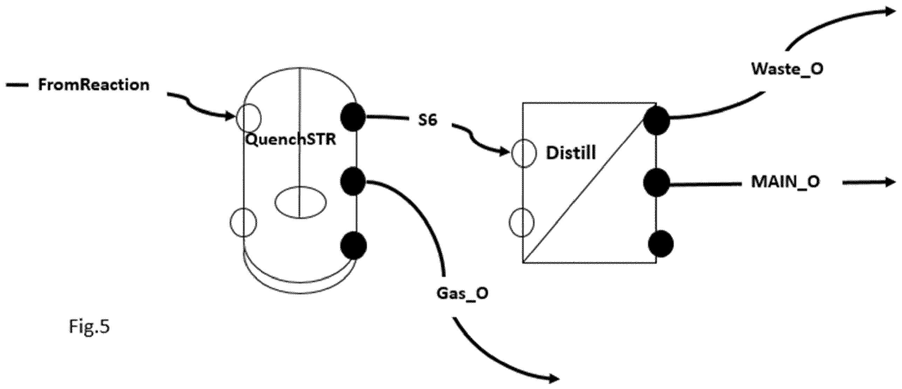

la Fig. 5 est un diagramme d'équipements du procédé de purification de la DVL par neutralisation thermique du peroxyde,
la Fig. 6 est un diagramme d'opérations du procédé de production sertraline-tetralone sans racémisation,
la Fig. 7 est un diagramme d'opérations du procédé de production sertraline-tetralone avec racémisation,
la Fig. 8 est un diagramme d'opérations du procédé de production sertraline-tetralone avec racémisation et recyclage de l'acétonitrile de racémisation,
la Fig. 9 est un diagramme d'équipements du procédé de production sertraline-tetralone sans racémisation,
la Fig. 10 est un diagramme d'équipements du procédé de production sertraline-tetralone avec racémisation et recyclage de l'acétonitrile de racémisation,
la Fig. 11 illustre de façon schématique un dispositif conforme à des modes de réalisation.

## Description détaillée de l'invention

D'une manière générale, les modes de réalisation de l'invention comportent les étapes décrites ci-après.

Etape 1: définition des matières premières et des espèces

1.1. Définition par l'utilisateur des matières premières entrant dans le procédé
1.2. Décomposition des matières premières en espèces chimiques (ex.: la matière première « alcool azéotropique » est décomposée en ses espèces chimiques la constituant, à savoir 96 % d'éthanol et 4 % d'eau)
1.3. Information par l'utilisateur des espèces chimiques ou des matières biologiques produites dans le procédé

Etape 2 : Schéma de blocs d'opération

2.1. Proposition par l'utilisateur ou création à partir d'une recette de laboratoire d'un schéma de blocs d'opération constitué de blocs d'opération. On dispose d'une bibliothèque BBOP de blocs d'opération BOP, chaque bloc d'opération présentant un nombre d'entrées et de sorties, en espèces, matières premières, ou énergie et représentant une opération élémentaire, telle qu'un mélange, une réaction, une séparation, un chauffage. Ces blocs d'opération BOP représentent des transformations des flux ou des quantités indépendamment du volume ou du temps que cela nécessite. Ces blocs d'opération BOP ne sont pas associés à des équipements et ne contiennent pas de notion de productivité.

2.2. Prise en compte par le système des relations entre les entrées et les sorties de chacun des blocs d'opération BOP sélectionnés. L'utilisateur peut fournir ces relations en extrayant un modèle d'une première bibliothèque de modèles d'opération MOP1 et en précisant éventuellement des paramètres de ce modèle, par exemple un ratio entre un débit d'entrée et de sortie du bloc. Ces relations peuvent concerner divers paramètres tels que des températures, des pressions, des quantités ou des flux de matière, des concentrations dans différentes phases. Il s'agit à ce stade d'un mode dit « Guess » en terminologie anglo-saxonne : seuls l'intuition, l'expérience de l'utilisateur sont utilisés, aucune information physico-chimique n'est nécessaire.

2.3. Au moyen du schéma de blocs d'opération et des relations entre les entrées et les sorties de chacun des blocs d'opération BOP sélectionnés, le système détermine des bilans globaux du procédé. Ces bilans globaux peuvent concerner la production des espèces chimiques ou des matières biologiques produites dans le procédé, la consommation de matières premières ou d'énergie. Cette détermination peut s'opérer indifféremment en continu (données de flux) ou en discontinu (données de quantité par lot). A ce stade, le système peut également déterminer une première estimation de critères de performance du procédé basée sur les consommations de matière premières et/ou d'énergie, ce critère pouvant être économique ou environnemental.

2.4. La possibilité est alors offerte à l'utilisateur de répéter l'étape 2.1, en sélectionnant d'autres blocs d'opération ou en les agençant autrement pour obtenir un autre schéma de blocs d'opération et/ou de répéter l'étape 2.2 en choisissant d'autres relations entre les entrées et les sorties des blocs d'opération sélectionnés, puis d'effectuer à nouveau l'étape 2.3 de détermination des bilans globaux, et de poursuivre jusqu'à obtention de bilans globaux satisfaisants.

2.5. Optionnellement, la possibilité est offerte à l'utilisateur à l'issue d'une étape 2.3 ou 2.4 de remplacer les relations entre les entrées et les sorties de certains des blocs d'opération BOP par des relations extraites d'une seconde bibliothèque de modèles d'opération MOP2. Cette seconde bibliothèque de modèles d'opération MOP2 comporte des caractéristiques plus élaborées, tenant compte par exemple d'informations thermodynamiques, ou de composition de phases. Le système détermine alors les bilans globaux, suivant l'étape 2.3, sur base des caractéristiques améliorées. L'utilisateur sélectionnera avantageusement les blocs d'opération pour lesquels il faut remplacer les relations entrées/sorties parmi les blocs d'opération les plus critiques dans le procédé. A ce stade, le système peut également proposer une deuxième détermination des critères de performance basée sur les consommations de matière premières et/ou d'énergie.

Etape 3 : Schéma d'équipements

3.1. Transformation du schéma de blocs d'opération en un schéma d'équipements représentant l'installation industrielle à concevoir. Des équipements sont choisis dans une bibliothèque d'équipements génériques BEQGEN ou dans une bibliothèque d'équipements spécifiques BEQSPEC pour réaliser les opérations d'un ou plusieurs blocs d'opération BOP du schéma de blocs d'opération, jusqu'à la transformation de tous les blocs d'opération du schéma de blocs d'opération. Les équipements de la bibliothèque BEQGEN sont des équipements génériques : leurs caractéristiques peuvent rester assez floues, voire idéalisées : un réacteur peut par exemple être défini comme un système adiabatique parfaitement mélangé, indépendamment des moyens pour parvenir à ce résultat. Les équipements de la bibliothèque BEQSPEC sont des équipements spécifiques. Ils peuvent alors avoir des caractéristiques bien précises et peuvent être associés à une référence constructeur donnée. La démarche présentée autorise également la création directe d'un schéma d'équipements sans création préalable d'un diagramme d'opérations. Un tel raccourci peut par exemple trouver son utilité si l'on cherche à représenter une installation déjà existante.

3.2. Spécification des différentes opérations réalisées dans chaque équipement. Pour chaque opération, on spécifie en particulier sa nature (chargement de matière première, réaction, vidange, changement de température, etc.) ainsi que sa durée. L'ensemble de ces spécifications d'équipements correspond à une procédure opératoire telle que celles utilisées dans les ateliers industriels. Cette procédure opératoire n'implique pas d'éléments de modélisation.

3.3. Détermination par le système de paramètres de fonctionnement de l'installation industrielle représentée par le schéma d'équipement à l'aide des bibliothèques de modèles MOP1 et MEQ1 ainsi que de la connectivité entre les équipements. Les modèles de la bibliothèque MEQ1 servent à décrire les opérations effectuées dans les équipements telles que saisies à l'étape 3.2. De même que pour la bibliothèque MOP1, ce sont des modèles basés sur une description macroscopique des phénomènes et ne faisant appel à aucune donnée physico-chimique. Les paramètres de fonctionnement de l'installation industrielle peuvent comprendre des productivités, des rendements, des paramètres de qualités des produits, tels que la pureté, la production de déchets, la consommation d'énergie. A ce stade, le système peut déterminer une première évaluation d'un critère de performance basé à la fois sur la consommation de matières premières, d'énergie, la taille et le mode d'opération des équipements. Ce critère peut par exemple être économique ou environnemental.

3.4. La possibilité est alors offerte à l'utilisateur de répéter l'étape 3.1, en sélectionnant d'autres équipements, et/ou de répéter l'étape 3.2 en choisissant d'autres caractéristiques pour les opérations réalisées dans les équipements, puis d'effectuer à nouveau l'étape 3.3 de détermination de paramètres de fonctionnement, et de poursuivre jusqu'à obtention de paramètres respectant les contraintes fixées.

3.5. Optionnellement, la possibilité est offerte à l'utilisateur à l'issue d'une étape 3.3 ou 3.4 de remplacer les caractéristiques de certaines opérations effectuées dans des équipements génériques ou spécifiques du schéma d'équipement par des caractéristiques extraites d'une seconde bibliothèque MEQ2 de modèles d'opérations effectuées dans des équipements. Les modèles issus de cette seconde bibliothèque sont nettement plus élaborés et permettent par exemple de calculer des conversions dans des réacteurs ou des performances de séparation en distillation à partir de données physico-chimiques. Le système détermine alors à nouveau des paramètres de fonctionnement de l'installation industrielle, suivant l'étape 3.3, sur base des caractéristiques améliorées. A ce stade, le système peut déterminer des critères de performance basée à la fois sur la consommation de matières premières, d'énergie, la taille et le mode d'opération des équipements.

3.6. Optionnellement, la possibilité est offerte à l'utilisateur à l'issue d'une étape 3.3 ou 3.4 de remplacer les caractéristiques de certaines opérations effectuées dans des équipements génériques ou spécifiques du schéma d'équipement par des caractéristiques extraites d'une troisième bibliothèque MEQ3 de modèles d'opérations effectuées dans des équipements. Les modèles issus de cette troisième bibliothèque sont des modèles détaillés et permettent par exemple de représenter des non-idéalités de mélange ou des phénomènes hydrodynamiques complexes via la prise en compte de telle ou telle caractéristique géométrique d'équipements spécifiés. Le système détermine alors à nouveau des paramètres de fonctionnement de l'installation industrielle, suivant l'étape 3.3, sur base des caractéristiques améliorées. A ce stade, le système peut déterminer des critères de performance basée à la fois sur la consommation de matières premières, d'énergie, la taille et le mode d'opération des équipements.

**Example 1**

Un exemple d'application de ce procédé de simulation est donné ci-après. Il est inspiré d'un cas réel et concerne une purification située en aval d'une synthèse organique qui produit de la delta-valerolactone. L'objectif est de minimiser le coût de production de la delta-valerotlactone. Cet objectif peut se décliner dans les deux questions suivantes :

- peut-on proposer des améliorations simples à la technologie de purification actuelle ?
- peut-on proposer d'autres technologies de purification avec des coûts inférieurs ? Comme exposé dans la section ci-dessous, l'information de départ est restreinte, ce qui empêche tout recours à des modèles élaborés (que ce soit en matière de thermodynamique ou de cinétique) à moins de recourir à des campagnes de mesures longues et coûteuses. L'approche descendante ici exposée consiste à réaliser une première série de calculs sur la base des informations disponibles ou accessibles immédiatement et gratuitement. Puis, sur la base de ces calculs, on identifie les informations supplémentaires réellement nécessaires afin de minimiser l'effort de collecte de cette information.

En fin de réaction, la delta-valerolactone, se trouve dans une solution aqueuse qui contient également de la cyclopentanone, du peroxyde d'hydrogène et de l'acide acétique. On cherche à retirer ces deux dernières espèces et à déshydrater le produit. La procédure traditionnelle consiste à :

- neutraliser le peroxyde avec du sulfite de sodium
- neutraliser l'acide acétique avec du carbonate de sodium
- extraire les molécules organiques à l'aide d'un solvant organique
- évaporer l'eau pour faire précipiter les sels
- filtrer les sels
- évaporer le solvant organique.

Nous envisagerons également une configuration basée sur la neutralisation thermique du peroxyde et le retrait de l'acide acétique par distillation.

Etape 1 : définition des matières premières et des espèces

Les espèces chimiques mises en jeu dans les procédés étudiés sont listées dans la Table 2. Certaines d'entre elles proviennent des matières premières listées dans la Table 1, d'autres sont générées par réaction chimique.
Pour chacune des espèces de la Table 2, sont fournies quelques informations que n'importe qui peut trouver gratuitement et rapidement.

**Table 1 : matières premières et espèces injectées dans les procédés de purification de la DVL**

| Matières premières | Espèces associées (% masse) |
|---|---|
| Mélange de réaction | 54,01% $H_2O$ ; 1,11 %CP ; 3,48% $H_2O_2$ ; 16,27% AcH ; 25,13% DVL |
| MIBK fraiche | 100% MIBK |
| Sulfite de sodium 20% | 80% $H_2O$ ; 20% $Na_2SO_3$ |
| Carbonate de sodium 20% | 80% $H_2O$ ; 20% $Na_2CO_3$ |
| **Soit 4 matières premières** | **Soit 8 espèces associées** |

**Table 2 : liste des espèces mises en jeu dans les procédés de purification de la DVL**

| Nom courant ou formule | CAS | Nom dans ce document | Masse molaire (g/mol) | Point d'ébullition normal (°C) |
|---|---|---|---|---|
| cyclopentanone | 120-92-3 | CP | 84.12 | 131 |
| delta-valerolactone | 542-28-9 | DVL | 100.12 | 230 |
| Eau | 7732-18-5 | $H_2O$ | 18.02 | 100 |
| $H_2O_2$ | 7722-84-1 | $H_2O_2$ | 34.01 | - |
| Acide acétique | 64-19-7 | AcH | 60.05 | 118 |
| Sulfite de Sodium | 7757-83-7 | $Na_2SO_3$ | 126.04 | - |
| Carbonate de Sodium | 497-19-8 | $Na_2CO_3$ | 105.99 | - |
| $CO_2$ | 124-38-9 | $CO_2$ | 44.01 | - |
| Acétate de Sodium | 127-09-3 | AcNa | 82.03 | - |
| Sulfate de Sodium | 7757-82-6 | $Na_2SO_4$ | 142.04 | - |
| MIBK | 108-10-1 | MIBK | 100.16 | 116 |
| Dioxygène | 7782-44-7 | $O_2$ | 32.00 | - |

Etape 2 : Schéma de blocs d'opération

2.1 Aspects généraux

Dans l'approche descendante que nous proposons, l'étape 2.1 pour la modélisation d'un processus est l'élaboration d'un schéma de blocs d'opération. Chaque icône de ce schéma représente une opération (c'est-à-dire une action sur un flux ou sur une quantité). A l'étape 2.2, ces opérations sont décrites par les relations entre leurs entrées et leurs sorties et peuvent être représentées par des modèles empiriques très simples ou par des modèles thermodynamiques plus précis. A l'étape 2.3, la résolution des équations de bilans associées aux relations entre les entrées et les sorties de chaque bloc et à la connectivité des blocs entre eux nous permettra de connaitre les flux (ou quantités) de masse et de chaleur à chaque endroit du système, et en particulier en sortie.
Les trois schémas d'opération considérés sont représentés :

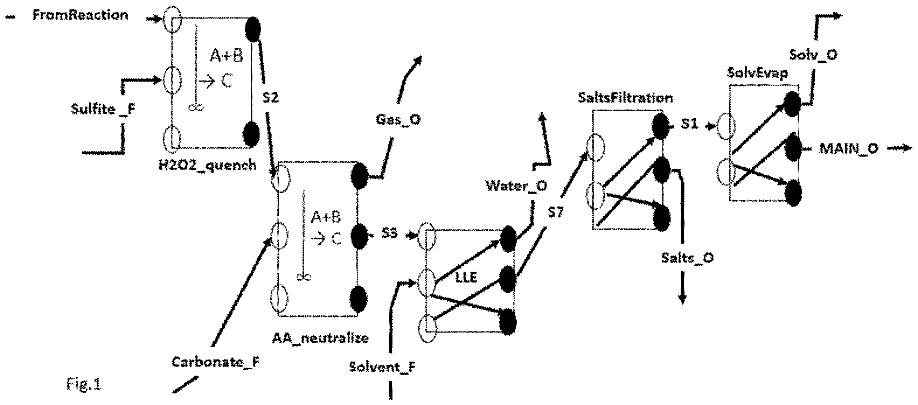

- Sur la figure 1 : schéma classique de purification par neutralisation saline et extraction liquide-liquide sans recyclage de solvant (cas 1)

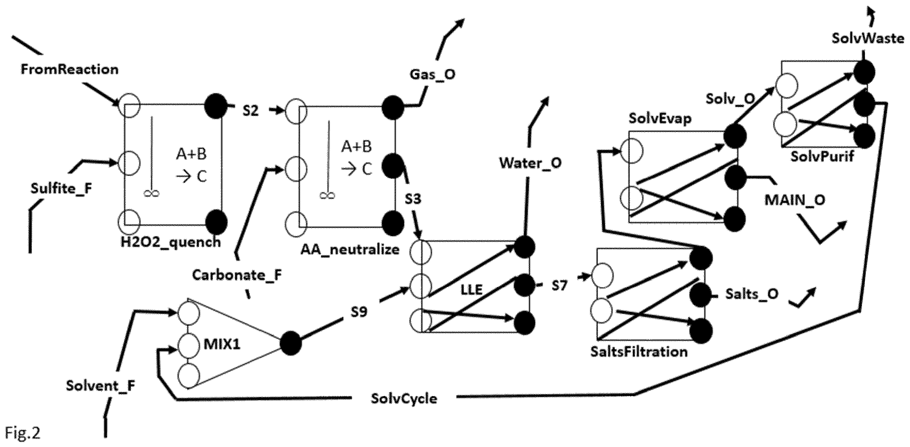

- Sur la figure 2 : schéma alternatif de purification par neutralisation saline et extraction liquide-liquide avec recyclage de solvant (cas 2)

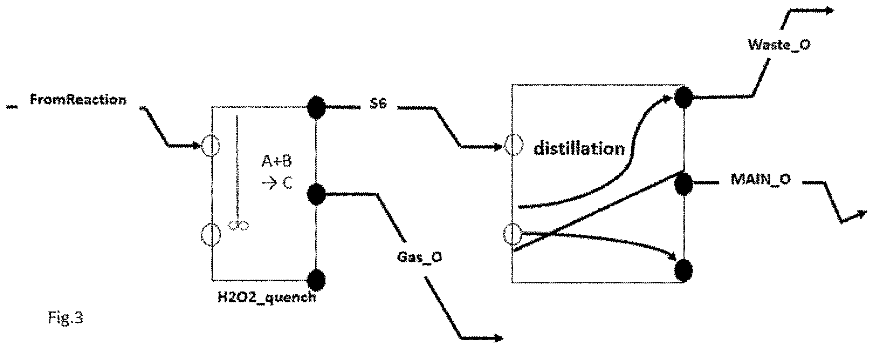

- Sur la figure 3 : schéma alternatif de purification par neutralisation thermique et distillation (cas 3)

Les schémas de blocs d'opération peuvent aussi bien se lire en flux (kg/h) pour les procédés continus qu'en quantité (kg) traitée par opération pour les procédés discontinus.

2.2 Définition des relations entre les entrées et les sorties des blocs d'opérations

Les modèles de la bibliothèque MOP1 proposent une description très macroscopique de l'effet de chaque bloc d'opération sur les flux ou quantités traversantes. Ce descriptif macroscopique se prête bien à l'utilisation d'hypothèses basées sur l'intuition et l'expérience.
Le schéma de la figure 1 comporte deux réactions *H2O2_quench* et *AA_neutralize* correspondant respectivement à la neutralisation de H2O2 par Na2SO3 et de AcH par Na2CO3. Ce schéma comporte également trois séparations *LLE, SaltsFiltration* et *SolvEvap* correspondant respectivement à l'extraction de la DVL et de la CP par un solvant organique (avec retrait de l'eau et précipitation des sels), la filtration des sels, l'évaporation du solvant.
Le schéma de la figure 2 est extrêmement similaire à celui de la figure 1 avec en plus l'opération *SolvPurif*-destinée à séparer le solvant à jeter de celui pouvant être recyclé - ainsi que l'opération *MIX1* destinée à mélanger le solvant frais et le solvant recyclé. Le schéma de la figure 3 comporte simplement le bloc réaction *H2O2_quench* correspondant à la neutralisation thermique de H2O2 et le bloc séparation *distillation* correspondant au retrait de H2O et AcH.
De façon générale, les opérations de mélange consistent simplement à regrouper les masses (ou débits massiques) de chaque espèce contenue dans l'ensemble des flux entrants. Pour les opérations de séparations et de réaction possédant plusieurs flux de sortie, un ratio est fourni par l'utilisateur pour chaque espèce afin d'indiquer sa répartition entre les flux de sortie. Dans les exemples ci-après ces ratios sont souvent de 0% ou 100% mais ils peuvent prendre n'importe quelle valeur entre 0% et 100%. Par ailleurs, ce système de ratios est ici illustré sur des situations à deux flux sortants mais il s'applique également sur des situations où ces flux seraient plus nombreux ainsi que sur des équilibres entre phases.
Les phénomènes réactifs représentés par les blocs de réactions sont décrits dans la Table 3.

**Table 3** : **modèles de réaction**

| Nom | Equation | Conversion |
|---|---|---|
| H2O2_quench (cas 1 et 2) | H2O2 + Na2SO3 → H2O + Na2SO4 | 100% de H2O2 |
| AA_neutralize | 2 AcH + Na2CO3 ----> 2 AcNa + H2O + CO2 | 100% de AcH |
| H2O2_quench (cas 3) | 2 H2O2 ----> 2 H2O + O2 | 100% de H2O2 |

La table ci-dessous comporte des ratios de partitions correspondant à l'hypothèse selon laquelle, lors de la neutralisation chimique de AcH, tout le CO2 généré est évacué sans entrainement de liquide. Dans la Table 9, une hypothèse identique est réalisée pour le dégagement de O2 dans le cas d'une neutralisation thermique.

|  | Gas_O | S3 |
|---|---|---|
| CO2 | 100% | 0% |
| Autres espèces | 0% | 100% |

**Table 4** : ratios de partition des espèces entre les sorties de *AA_neutralize*

Les ratios du tableau ci-dessous représentent une extraction liquide-liquide où les phases aqueuses et organiques sont totalement immiscibles. Pour le retrait de l'eau, toute la charge de l'opération est portée à 100°C, l'eau est ainsi évaporée (enthalpie de changement d'état de 2260 kJ/kg). Puis tous les flux sortants sont ramenés à 25°C à l'état liquide.

**Table 5** : ratios de partition des espèces entre les sorties de *LLE*

|  | Water_O | S7 |
|---|---|---|
| H2O, CO2 | 100% | 0% |
| Autres espèces | 0% | 100% |

Pour le retrait des sels (cas 1 et 2), on suppose une filtration sans rétention de liquide dans le solide retenu.

**Table 6** : ratios de partition des espèces entre les sorties de *SaltsFiltration*

|  | Salts_O | S1 |
|---|---|---|
| Na2SO3, Na2CO3, AcNa, Na2SO4 (solides) | 100% | 0% |
| Autres espèces | 0% | 100% |

Pour l'opération *SolvEvap,* on considère que toute la charge est portée à 120°C et que la MIBK est ainsi évaporée puis que les deux flux sortants sont ramenés à 25°C sous état liquide. L'enthalpie de changement d'état est prise égale à 400 kJ/kg.

**Table 7** : ratios de partition des espèces entre les sorties de *SolvEvap*

|  | Solv_O | MAIN_O |
|---|---|---|
| MIBK | 100% | 0% |
| CP, DVL | 0% | 100% |
| Autres espèces | 50% | 50% |

Dans le procédé de la figure 2, on considère qu'une partie du solvant ne peut pas être réutilisée et que le recyclage concerne 95% du solvant (voir Table 8). De même que pour *SolvEvap,* on considère une évaporation à 120°C du flux de MIBK.

**Table 8** : ratios de partition des espèces entre les sorties de *SolvPurif*

|  | SolvWaste | SolvCycle |
|---|---|---|
| MIBK | 5% | 95% |
| Autres espèces | 100% | 0% |

**Table 9** : ratios de partition des espèces entre les sorties de *H2O2_quench* dans le cas de la neutralisation thermique

|  | Gas_O | S6 |
|---|---|---|
| O2 | 100% | 0% |

(suite)

|  | Gas_O | S6 |
|---|---|---|
| Autres espèces | 0% | 100% |

**Table 10** : **ratios de partition des espèces entre les sorties de** *distillation*

|  | Waste_O | MAIN_O |
|---|---|---|
| O2, H2O, AcH | 100% | 0% |
| CP, CVL, AcNa | 0% | 100% |
| H2O2 | 0% | 100% |

Le système de ratios de partition est dans le cas présent utilisé pour rendre compte de séparation généralement idéales. De par sa structure, ce système permet de garder conscience de cette hypothèse d'idéalité et autoriserait à représenter des écarts à l'idéalité, par exemple en prenant des ratios 97%/3% au lieu des 100%/0% actuels.

2.3 Détermination des bilans globaux

Dans les trois configurations, on cherche à traiter une charge de 4050 kg de mélange réactionnel (voir composition dans la Table 1).

Les débits de sulfite et de carbonate sont calculés de façon à neutraliser totalement H2O2 et AcH. Le débit de MIBK fraiche est calculé de façon à ce que dans *LLE,* on mélange 4000 kg de MIBK avec la charge de DVL et de CP.

Dans le cas de base, on obtient le bilan de matière de la Table 11 et le bilan d'énergie de la Table 12.

**Table 11** : **description des flux du procédé dans le cas de base (cas 1)**

|  | Flux | Matières premières | Quantités |
|---|---|---|---|
| SORTANTS | *MAIN_O* |  | 1063 kg |
|  | *Gas_O* |  | 242 kg |
|  | *Water_O* |  | 6921 kg |
|  | *Salts_O* |  | 1525 kg |
|  | *Solv_O* |  | 4000 kg |
| INTRANTS | *FromReaction* | Mélange de réaction | 4050 kg |
|  | *Sulfite_F* | Sulfite de sodium 20% | 2700 kg |
|  | *Carbonate_F* | Carbonate de sodium 20% | 3000 kg |
|  | *Solvent_F* | MIBK fraiche | 4000 kg |
| INTERNES | *S2* |  | 6750 kg |
|  | *S3* |  | 9509 kg |
|  | *S7* |  | 6599 kg |
|  | *S1* |  | 5063 kg |

**Table 12** : **échanges thermiques dans le cas de base (cas 1)**

| Bloc opération | Sens de l'échange | Energie (kWh) |
|---|---|---|
| *H2O2_quench* | Apport au procédé | 259 |
| *AA_neutralize* | Apport au procédé | 68 |
| *LLE* | Apport au procédé | 4808 |

(suite)

| Bloc opération | Sens de l'échange | Energie (kWh) |
|---|---|---|
| | Retrait du procédé | 5197 |
| *SolvEvap* | Apport au procédé | 678 |
| | Retrait du procédé | 678 |

Dans le cas avec recyclage du solvant d'extraction, on obtient les résultats de la Table 13 et de la Table 14. Comparativement au cas de base, les principales différences interviennent au niveau des entrées et sorties de solvant. Le recyclage permet de les réduire considérablement moyennant un apport d'énergie supplémentaire.

**Table 13 : description des flux du procédé dans le cas avec recyclage du solvant d'extraction (cas 2)**

| | Flux | Matières premières | Quantités |
|---|---|---|---|
| SORTANTS | *MAIN_O* | | 1063 kg |
| | *Gas_O* | | 242 kg |
| | *Water_O* | | 6921 kg |
| | *Salts_O* | | 1525 kg |
| | *SolvWaste* | | 200 kg |
| INTRANTS | *FromReaction* | Mélange de réaction | 4050 kg |
| | *Sulfite_F* | Sulfite de sodium 20% | 2700 kg |
| | *Carbonate_F* | Carbonate de sodium 20% | 3000 kg |
| | *Solvent_F* | MIBK fraiche | 200 kg |
| INTERNES | *S2* | | 6750 kg |
| | *S3* | | 9509 kg |
| | *S7* | | 6599 kg |
| | *S1* | | 5063 kg |
| | *Solv_O* | | 4000 kg |
| | *SolvCycle* | | 3800 kg |
| | *S9* | | 4000 kg |

**Table 14 : échanges thermiques dans le cas du procédé avec recyclage du solvant d'extraction (cas 2)**

| Bloc opération | Sens de l'échange | Energie (kWh) |
|---|---|---|
| *H2O2_quench* | Apport au procédé | 259 |
| *AA_neutralize* | Apport au procédé | 68 |
| *LLE* | Apport au procédé | 4808 |
| | Retrait du procédé | 5197 |
| *SolvEvap* | Apport au procédé | 678 |
| | Retrait du procédé | 678 |
| *SolvPurif* | Apport au procédé | 600 |
| | Retrait du procédé | 600 |

Dans le cas du procédé avec neutralisation thermique du peroxyde, on obtient le bilan matière de la Table 15 et le bilan d'énergie de la Table 16. Les quantités de matière et d'énergie sont sensiblement inférieures à celles des autres configurations. En effet, on n'apporte ni solvant d'extraction, ni eau (avec le sulfite et le carbonate).

**Table 15 : description des flux du procédé dans le cas de la neutralisation thermique du peroxyde (cas 3)**

|  | Flux | Matières premières | Quantités |
|---|---|---|---|
| SORTANTS | *MAIN_O* |  | 1063 kg |
|  | *Gas_O* |  | 66 kg |
|  | *Waste_O* |  | 2921kg |
| INTRANTS | *FromReaction* | Mélange de réaction | 4050kg |
| INTERNES | *S6* |  | 3984 kg |

**Table 16** : **échanges thermiques dans le cas de la neutralisation thermique du peroxyde (cas 3)**

| Bloc opération | Sens de l'échange | Energie (kWh) |
|---|---|---|
| *H2O2_quench* | Apport au procédé | 315 |
| *distillation* | Apport au procédé | 1883 |
|  | Retrait du procédé | 2157 |

Ces six tableaux constituent trois résultats de simulation.

2.4 Utilisation d'une recette de laboratoire

Lorsque l'on dispose d'une recette de laboratoire - c'est-à-dire d'une procédure expérimentale éventuellement accompagnée d'éléments de résultats - le programme d'ordinateur sur lequel porte cette invention permet la saisie des informations présentes dans la recette. Il est possible de saisir toutes les informations de la recette sans préjuger de leur éventuelle utilisation ultérieure ; en parallèle aucune information ne doit impérativement être fournie hormis la nature de chaque opération. Une fois cette recette saisie, le programme d'ordinateur est capable d'en interpréter le contenu pour créer la structure d'un diagramme de blocs-opérations sur la base de ce contenu.
La recette de laboratoire suivante peut-être utilisée comme base pour créer le diagramme d'opérations de la figure 1.

- Ajouter 4,05 kg de *mélange de réaction* et 2,75 kg de *sulfite de sodium 20%.* Laisser réagir durant 2 heures
- Ajouter 3,5 kg de *carbonate de sodium 20%,* laisser réagir durant 1 heure. Un dégagement gazeux est observé
- Ajouter 4 kg de *MIBK fraiche.* Mélanger et laisser décanter. Evaporer la phase aqueuse à 100°C. Une précipitation est observée.
- Filtrer le mélange
- Concentrer par évaporation du solvant à 120°C

Une recette de laboratoire peut par exemple provenir d'un article scientifique relatif à une expérience de synthèse. Cet article constituant alors l'un des premières sources d'information de la démarche.

2.5 Evaluation des coûts variables

Les bilans des six tableaux précédents vont à présent servir de base à des calculs économiques, ils permettent de calculer la part variable du coût de production. Ces coûts seront rapportés au kilogramme de DVL dans le flux *MAIN_O.*
Les hypothèses utilisées sont exposées ci-dessous. Pour beaucoup, ce sont des ordres de grandeur issus de l'expérience.

**Table 17** : **coût unitaire des matières premières**

| Matière première | Prix (€/kg) |
|---|---|
| Mélange de réaction | 7.00 |
| Sulfite de sodium 20% | 0.38 |

(suite)

| Matière première | Prix (€/kg) |
|---|---|
| Carbonate de sodium 20% | 0.25 |
| MIBK fraiche | 0.98 |

**Table 18 : coût de traitement des sorties secondaires de matière**

| Flux | Coût de traitement (€/kg) |
|---|---|
| Gas_O (tous les cas) | 0.00 |
| Water_O (cas 1 et 2) | 0.15 |
| Salts_O (cas 1 et 2) | 0.10 |
| Solv_O (cas 1) | 0.15 |
| SolvWaste (cas 2) | 0.15 |
| Waste_O (cas 3) | 0.15 |

Les flux d'énergie apportés aux procédés (chauffage/évaporation) se voient affecter un coût de 0.10 €/kWh. Les flux d'énergie retirés des procédés (refroidissement/condensation) se voient affecter un coût de 0.05 €/kWh.

Note : les bilans de matière portent sur des espèces, l'économie sur la valeur des matières premières d'où l'importance de l'étape 1.2.

Sur la base des bilans de matière et d'énergie ainsi que des hypothèses économiques ci-dessus, on aboutit aux coûts variables des trois versions du procédé tels qu'exposés dans la Table 19.

**Table 19 : contribution de chaque entrée à la partie variable du coût de production de DVL. Les valeurs sont exprimées en €/kg par kg de DVL dans le flux *MAIN_O***

| | Procédé 1 | Procédé 2 | Procédé 3 |
|---|---|---|---|
| | Cas de base sans recyclage | Recyclage du solvant d'extraction | Neutralisation thermique |
| Mélange de réaction | 27.86 | 27.86 | 27.86 |
| Sulfite de sodium 20% | 1.01 | 1.01 | - |
| Carbonate de sodium 20% | 0.74 | 0.74 | - |
| MIBK fraiche | 3.85 | 0.19 | - |
| Traitement d'effluents | 1.76 | 1.20 | 0.43 |
| Energie | 0.86 | 0.95 | 0.32 |
| TOTAL | 36.07 | 31.94 | 28.61 |
| Ecart au cas de base | - | -11,4% | -20,7% |

On remarque que le procédé 2 permet, grâce au recyclage de solvant, des économies intéressantes à la fois au niveau de la MIBK fraiche et du traitement d'effluents. La contrepartie au niveau de l'énergie est minime.

Le procédé 3 permet des économies encore plus importantes car la technique de neutralisation employée réduit nettement les quantités de matières premières à injecter et les quantités de solvants (eau y compris) à séparer et retraiter.

Si l'on compare les procédés 1 et 2, on constate un net écart de coûts variables. Or, sachant que les deux configurations sont extrêmement proches, on peut supposer que le dispositif permettant le recyclage n'induira pas un supplément de coûts fixes suffisamment significatif pour compenser l'avantage du procédé 2 en matière de coûts variables. On peut donc considérer que le procédé 1 sera nécessairement moins compétitif que le procédé 2, il ne sera donc plus considéré pour la suite de l'étude.

Si l'on compare les procédés 2 et 3, on constate que le procédé 3 est nettement plus avantageux en matière de coûts variables. Toutefois, au vu des différences fondamentales (nature des réactions chimiques, structure du procédé, etc.) entre ces deux procédés, il apparaît nécessaire, pour tirer une conclusion sérieuse, de prendre en compte les coûts fixes et, à travers eux, les notions de temps et d'équipement.

Etape 3 : schéma d'équipements

### 3.1 Transformation en schéma d'équipements

Dans l'étape 3.1, chacun des blocs d'opération de deux schémas conservés est affecté à un équipement. Notons que plusieurs opérations peuvent être réalisés dans un même équipement (par exemple les deux réactions de neutralisation dans le cas du procédé 2).

Le système dispose d'une bibliothèque d'équipements génériques BEQGEN et d'une bibliothèque d'équipement spécifiques BEQSPEC, chaque équipement ayant ses caractéristiques.

Dans le cas présent (voir Table 20 et Table 21), tous les équipements concernés proviennent de la bibliothèque BEQGEN. Dans le cas des réacteurs, un premier choix technologique est effectué puisque l'on décide d'employer des réacteurs agités, choix très classique dans le cas de procédés discontinus. Dans le cas des séparateurs, on utilise pour l'instant des séparateurs génériques. Ainsi, il n'est pas nécessaire de prendre d'emblée position sur des questions telles que la nature du filtre ou le nombre de plateaux de la colonne de distillation. Nous sommes toutefois conscients que le choix effectué limite pour l'instant la précision des résultats relatifs aux équipements de séparation.

Une première valeur de volume (un peu arbitraire) est donnée pour chaque équipement. Celle-ci pourra être modifiée par la suite.

**Table 20 : affectation des opérations en équipements dans le cas du procédé 2 (recyclage du solvant d'extraction)**

| Bloc d'opération | Equipement | | |
|---|---|---|---|
| | Nom | Type | Volume ($m^3$) |
| H2O2_quench | QuenchSTR | Réacteur agité | 14 |
| AA_neutralize | | | |
| LLE | LL_extractor | Séparateur générique | 18 |
| SaltsFiltration | SaltsFilter | Séparateur générique | 10 |
| SolvEvap | Evaporator | Séparateur générique | 8 |
| SolvPurif | SolvPurif | Séparateur générique | 6 |
| MIX1 | SolventTank | Cuve | 6 |

**Table 21 : affectation des opérations en équipements dans le cas du procédé 3 (neutralisation thermique)**

| Bloc d'opération | Equipement | | |
|---|---|---|---|
| | Nom | Type | Volume ($m^3$) |
| H2O2_quench | QuenchSTR | Réacteur agité | 6 |
| distillation | Distill | Séparateur générique | 6 |

### 3.2 Définition des opérations effectuées sur chaque procédé

Au démarrage du procédé 2 (extraction liquide-liquide avec recyclage de solvant), l'équipement *SolventTank* contient 3800 kg de MIBK. Ensuite la procédure opératoire appliquée est celle exposée dans la Table 22. Pour un certain nombre d'opérations, une durée arbitraire de 0,01h est appliquée car on présume que la durée de ces étapes est négligeable au regard des durées de réactions et de séparations.

**Table 22 : procédure opératoire du procédé 2 (extraction liquide-liquide avec recyclage du solvant)**

| Ordre | Opération | Durée (h) |
|---|---|---|
| 1 | Chargement de 4050 kg de *Mélange réactionnel* dans *QuenchSTR* | 0,01h |
| 2 | Chargement de 2750 kg de *Sulfite de sodium 20%* dans *QuenchSTR* | 0,01h |
| 3 | Réaction dans *QuenchSTR* | 2h |
| 4 | Chargement de 3500 kg de *Carbonate de sodium 20%* dans *QuenchSTR* | 0,01h |
| 5 | Réaction dans *QuenchSTR* | 1h |
| 6 | Vidange partielle de *QuenchSTR* par *Gas_O* | 0,01h |
| 7 | Vidange totale de *QuenchSTR* dans *LL_extractor* | 0,01h |
| 8 | Chargement de 200 kg de *MIBK fraiche* dans *SolventTank* | 0,01h |
| 9 | Vidange totale de *SolventTank* dans *LL_extractor* | 0,01h |
| 10 | Agitation dans *LL_extractor* | 4h |
| 11 | Vidange partielle de *LL_extractor* par *Water_O* | 1h |
| 12 | Vidange totale de *extractor* dans *SaltsFilter* | 0,01h |
| 13 | Vidange partielle de *SaltsFilter* dans *Evaporator* | 2h |
| 14 | Vidange totale de *SaltsFilter* par *Salts_O* | 0,01h |
| 15 | Vidange partielle de *Evaporator* dans *SolvPurif* | 2h |
| 16 | Vidange partielle de *SolvPurif* dans *SolventTank* | 2h |
| 17 | Vidange totale de *Evaporator* par *MAIN_O* | 0,01h |
| 18 | Vidange totale de *SolvPurif* par *SolvWaste* | 0,01h |

Pour le procédé 3 (neutralisation thermique), la procédure opératoire est exposée dans la Table 30.

**Table 23** : **procédure opératoire du procédé 2 (extraction liquide-liquide avec recyclage du solvant)**

| Ordre | Opération | Durée (h) |
|---|---|---|
| 1 | Chargement de 4050 kg de *Mélange réactionnel* dans *QuenchSTR* | 0,01h |
| 2 | Chauffage à 80°C de *QuenchSTR* | 2h |
| 3 | Réaction dans *QuenchSTR* | 22h |
| 4 | Vidange partielle de *QuenchSTR* par *Gas_O* | 0,01h |
| 5 | Vidange totale de *QuenchSTR* dans *Distill* | 0,01h |
| 6 | Vidange totale de *Distill* par *Waste_O* et *MAIN_O* | 5h |

3.3 Détermination par le système des paramètres de fonctionnement suivant des modèles quess (MEQ1)

Les modèles de la bibliothèque MEQ1 servent à décrire les opérations se déroulant dans les équipements (voir Table 22 et Table 23). Ils sont extrêmement similaires dans leur principe et leur structure à ceux de la bibliothèque MOP1.
Pour les opérations de réaction (opérations 3 et 5 pour le procédé 2, opération 3 pour le procédé 3), les modèles utilisés sont strictement identiques à ceux de la Table 3.
Pour l'opération 6 du procédé 3 (la distillation de l'eau et de l'acide acétique), on utilise à l'identique le modèle décrit dans la Table 10. Les autres séparations représentées sur le diagramme d'opérations du procédé 2 sont transcrites sous forme de binômes {vidange partielle + vidange totale}. Pour les vidanges partielles, les ratios de partitions entre la matière extraite et la matière restant dans l'équipement sont ceux des tables 4 à 8. Ce qui revient de fait à conserver ces ratios pour la partition des espèces entre les flux de sortie.

Le passage du diagramme d'opérations au diagramme d'équipement ne demande donc pas un gros supplément d'informations tant que l'on utilise des équipements de la bibliothèque BEQGEN et des modèles d'opérations de la bibliothèque MEQ1. Le supplément d'information consiste essentiellement à donner des durées aux opérations.

Dans les deux cas, les bilans de matières et énergies entrantes et sortantes obtenues par le calcul sont exactement identiques à ceux obtenus avec les diagrammes d'opérations (voir tables 13 à 16).

Le calcul permet également de disposer du taux de remplissage de chaque équipement susceptible de stocker un contenu ainsi que du temps total de la séquence opératoire (voir Table 24 et Table 25). Dans le cas du procédé 2, on constate que certains volumes initialement spécifiés sont à changer afin d'aboutir à des taux d'occupation raisonnables. La méthodologie ici exposée permet un tel processus itératif.

**Table 24 : éléments de résultat pour la simulation du diagramme d'équipement du procédé 2**

| Donnée | Valeur | Volume | |
| --- | --- | --- | --- |
| | | Ancien | Nouveau |
| Taux d'occupation de *QuenchSTR* | 67% | 14 m$^3$ | 12 m$^3$ |
| Taux d'occupation de *LL_extractor* | 94% | 18 m$^3$ | 22 m$^3$ |
| Taux d'occupation de *SaltsFilter* | 82% | 10 m$^3$ | 10 m$^3$ |
| Taux d'occupation de *Evaporator* | 93% | 8 m$^3$ | 10 m$^3$ |
| Taux d'occupation de *SolvPurif* | 102% | 6 m$^3$ | 8 m$^3$ |
| Taux d'occupation de *SolventTank* | 97% | 6 m$^3$ | 8 m$^3$ |
| Temps Total | 14,1 h | | |

**Table 25 : éléments de résultat pour la simulation du diagramme d'équipement du procédé 3**

| Donnée | Valeur |
| --- | --- |
| Taux d'occupation de *QuenchSTR* | 67,5% |
| Taux d'occupation de *Distill* | 66,4% |
| Temps total | 29,03 h |

A ce stade, nous disposons des éléments d'information nécessaires pour procéder à un calcul de coûts fixes.

Les hypothèses relatives aux coûts variables restent d'actualité. Celles relatives aux coûts fixes sont exposées ci-dessous.

Pour le calcul du coût d'investissement d'un équipement, on se réfère à une taille et à un prix de référence selon la formule suivante :

$$\text{Prix de l'équipement (k€)} = \text{Prix de référence (k€)} \left( \frac{\text{taille}}{\text{Taille de référence}} \right)^{\text{élasticité}} \qquad (1)$$

Le détail des CAPEX des deux procédés étudiés apparaît dans la Table 26 et la Table 27.

On considère que le coût de l'équipement est amorti sur 64 000 heures. Nous considérons également un coût de maintenance de 5% du CAPEX par année de 8 000 heures.

Dans les deux cas, on considère un temps d'arrêt du procédé de 2,4 heures à chaque cycle. Lorsque le procédé est en fonctionnement, on considère que chaque équipement (excepté le stockage de solvant) mobilise un équivalent temps plein coutant 1 000 €/jour.

On considère un coût de contrôle de qualité de 100 000 €/an et un coût de frais généraux de 25% des autres coûts fixes.

**Table 26 : taille, paramètres économiques et coût de chaque équipement pour le procédé 2**

| Equipement | Taille | Taille de ref. | Prix de ref. (k€) | Elasticité | Prix de l'équipement (k€) |
|---|---|---|---|---|---|
| *QuenchSTR* | 12 m$^3$ | 3 m$^3$ | 1 000 | | 2 297 |
| *LL_extractor* | 22 m$^3$ | | | | 4 958 |
| *SaltsFilter* | 10 m$^3$ | 3 m$^3$ | 1 500 | 0.6 | 3 089 |
| *Evaporator* | 10 m$^3$ | | | | 3 089 |
| *SolvPurif* | 8 m$^3$ | | | | 2 702 |
| *SolventTank* | 8 m$^3$ | 3 m$^3$ | 100 | | 180 |
| Total CAPEX | | | | | 16315 |

**Table 27 : taille, paramètres économiques et coût de chaque équipement pour le procédé 2**

| Equipement | Taille | Taille de ref. | Prix de ref. (k€) | Elasticité | Prix de l'équipement (k€) |
|---|---|---|---|---|---|
| *QuenchSTR* | 6 m$^3$ | 3 m$^3$ | 1 000 | 0.6 | 1 516 |
| *Distill* | 6 m$^3$ | 3 m$^3$ | 1 500 | | 2 274 |
| Total CAPEX | | | | | 3 789 |

Il y a lieu de signaler que, dans la continuité du recours aux séparateurs génériques, on fait l'hypothèse que tous ces séparateurs ont les mêmes paramètres de coûts d'investissement. Il s'agit évidemment d'une hypothèse forte mais elle nous permet à ce stade une économie de temps et d'informations très importante.

**Table 28 : coût total de production (en € par kg de DVL dans *MAIN_O*) avec ventilation des coûts fixes**

| Poste | Procédé 2 | Procédé 3 |
|---|---|---|
| Amortissement CAPEX | 4.13 | 1.83 |
| Maintenance | 1.65 | 0.73 |
| Main d'oeuvre | 2.89 | 2.38 |
| Contrôle qualité | 0.20 | 0.39 |
| Frais généraux | 2.22 | 1.33 |
| Sous-total coûts fixes | 11.09 | 6.65 |
| Sous-total coûts variables | 31.94 | 28.61 |
| Total général | 43.03 | 35.26 |

Nous pouvons constater que les coûts fixes amplifient l'avantage du procédé 3 (basé sur la neutralisation thermique du peroxyde) par rapport au procédé 2 (basé sur la neutralisation saline). C'est donc sur le procédé 3 uniquement qu'il est judicieux de focaliser la suite des investigations.

Concernant le choix de recourir aux séparateurs génériques, on peut constater que, même si les contributions liées à l'équipement étaient réduites à 0 €/kg dans le cas du procédé 2, le procédé 3 resterait plus avantageux. Le choix des séparateurs génériques et l'hypothèse associée n'ont donc pas faussé la conclusion comparative.

Si ce choix n'avait pas été fait, il aurait fallu fournir une grosse quantité d'informations sur les séparateurs du procédé 2 pour ensuite se rendre compte que ce procédé était à abandonner.

À ce stade de l'étude, nous n'avons eu recours à aucun paramètre cinétique et extrêmement peu de données thermodynamiques. Par ailleurs, seule la séparation par neutralisation thermique du peroxyde et distillation de l'acide acétique est encore considérée.

En d'autres termes, du point de vue des paramètres liés aux espèces, on peut dire la chose suivante :

- une approche ascendante (« bottom-up ») aurait nécessité d'emblée de collecter d'abondantes données sur chacune des 12 espèces de la Table 1. Ainsi que sur la façon dont elles peuvent interagir.

- l'approche descendante (« top-down ») ici exposée nous permet de réduire la liste d'espèces à 6 entrées (dont aucune espèce saline) avant toute collecte de données physico-chimiques, excepté des masses molaires et températures normales d'ébullition.

De la même façon, l'approche montante aurait nécessité une étude des réactions de neutralisation du peroxyde par le sulfite et de neutralisation de l'acide acétique par le carbonate. L'approche descendante, rend cette étude inutile.

Les modèles BOP1 et MEQ1 utilisés jusqu'ici nous ont permis un tri entre les configurations moyennant un investissement modeste en termes de temps et d'argent. Toutefois, pour la configuration qui apparaît comme la meilleure, ces modèles ne remplacent pas une étude avec des modèles conventionnels de génie chimique (bibliothèque MEQ2).

A ce stade, nous avons tiré le maximum de la méthode GUESS. Il est maintenant temps de s'intéresser aux points faibles ou aux imprécisions qui peuvent impacter les performances du procédé.

3.4 Détermination par le système de paramètres de fonctionnement suivant un modèle standard (MEQ2)

La prochaine étape de l'étude amènerait trois axes de perfectionnement :

- une description de la cinétique de la neutralisation thermique du peroxyde, ce qui permettra de statuer sur la durée nécessaire pour cette réaction
- le choix d'un équipement de distillation, ce qui permettra d'évaluer plus finement les coûts d'investissement
- une description de la distillation par des lois d'équilibre thermodynamique, ce qui permettra d'évaluer plus finement la qualité de la séparation.

3.5 Détermination par le système de paramètres de fonctionnement suivant un modèle détaillé (MEQ3)

Il serait possible de raffiner encore l'étude en recourant à des modèles de la bibliothèque MEQ3. Ces modèles nécessitent de s'appuyer sur des équipements de la bibliothèque BEQSPEC car ils font appel à la description géométrique des équipements afin de statuer par exemple sur des non-idéalités de mélange ou la formation de points chauds.

**Example 2**

Un autre exemple d'application du procédé de simulation suivant l'invention est donné ci-après, en référence au procédé décrit dans le brevet US 6 444 854.

L'objectif général est de minimiser le coût de production de sertraline-tétralone énantiomériquement pure ou optiquement enrichie (en forme R) à partir d'un mélange contenant deux énantiomères (R et S) au moyen d'une chromatographie telle que la chromatographie sur lit mobile simulé (LMS)

Cet objectif général est décliné en une série de questions :

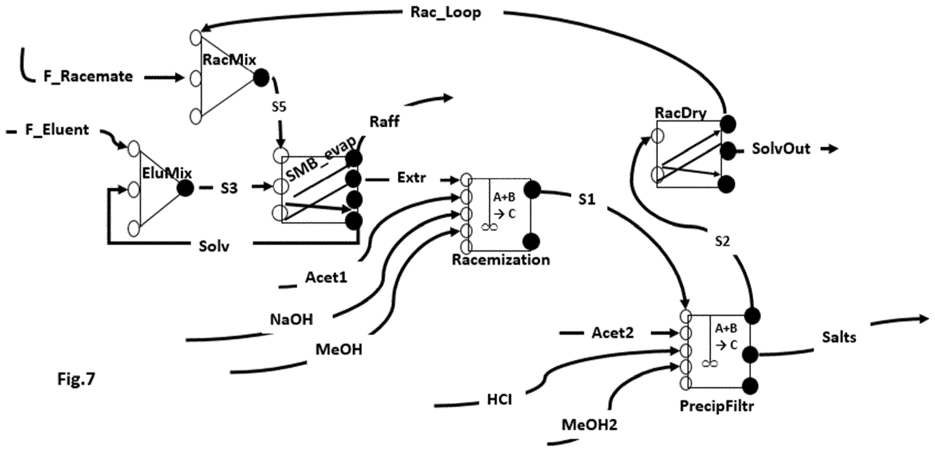

- est-il rentable de recycler les énantiomères indésirables dans un réacteur de racémisation pour mélange du racémate ainsi obtenu avec le produit d'injection frais (voir figure 7)?
- quels éléments du processus nécessitent une étude avancée pour assurer une bonne estimation des coûts et ensuite des choix techniques pertinents ? En d'autres termes, où sont les « goulots d'étranglement » en matière d'information ?

Contraintes

Les seules informations disponibles sur le procédé étudié proviennent du brevet précité. Ce document ne fournit aucune information fondamentale sur :

- la thermodynamique générale,
- les équilibres de sorption,
- la cinétique de la réaction de racémisation.

Les informations disponibles consistent en une courte série de rapports d'expériences indiquant ce qui a été injecté dans quel équipement et fournissent quelques informations macroscopiques sur les conséquences observées. Les informations contenues dans ce document pourraient aussi contenir certaines erreurs et doivent être traitées avec précautions.

Une approche classique ascendante (« bottom-up ») d'optimisation nécessiterait une longue et coûteuse campagne d'expériences pour collecter les informations fondamentales manquantes (thermodynamique, sorption, cinétique de la réaction de racémisation en particulier) pour la description de la chromatographie LMS et du réacteur de racémisation à travers des modèles classiques d'ingénierie des procédés.

Cette approche serait beaucoup trop complexe, longue et coûteuse à mettre en place dans le cadre d'une première évaluation.

Nous appliquons ci-après une approche descendante (« top-down ») suivant l'invention. Pour illustration, cette approche est utilisée pour comparer deux exemples de schéma de procédé :

- un schéma sans recyclage de l'énantiomère S, et sans racémisation :
- un schéma simple avec recyclage de l'énantiomère S, et avec racémisation :

Etape 1: définition des matières premières et des espèces

Pour le schéma sans recyclage, les seules matières premières sont le mélange racémique et le solvant frais. En cas de racémisation, il convient d'ajouter de l'acétonitrile, du méthanol, de la soude et de l'acide chlorhydrique.

Au final, les matières premières utilisées et les espèces associées sont listées dans la Table 29. A ces 8 espèces injectées, s'ajoute le NaCl qui peut être généré dans le procédé

**Table 29 : matières premières et espèces associées**

| Matières premières | Espèces associées |
|---|---|
| Sertraline-tétralone racémate | R et S |
| Solvant frais | 10 % MeOH et 90 % CH3CN |
| Acétonitrile | CH3CN |
| Méthanol | MeOH |
| NaOH | Na+ ; OH- |
| HCl | H+ ; Cl- |
| **Soit 6 matières premières** | **Soit 8 espèces** |

Etape 2 : Schéma de blocs d'opération

Dans le cadre de l'approche descendante proposée, la représentation des procédés commence par l'établissement de diagrammes d'opérations (étape 2.1).

Les deux schémas d'opération considérés sont représentés :

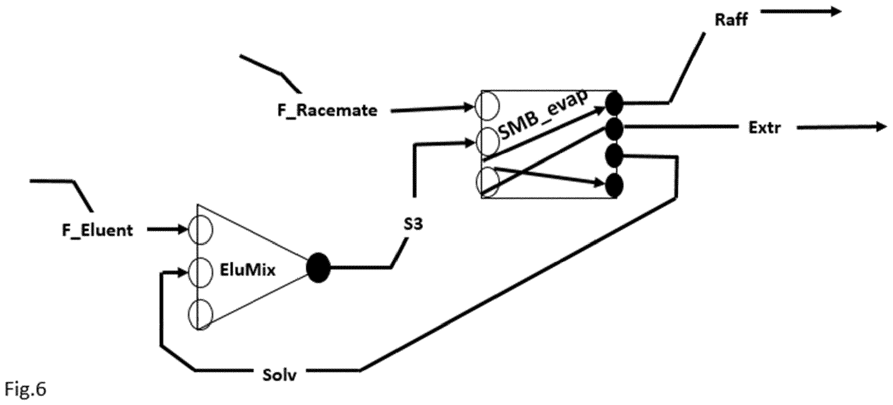

- Sur la figure 6 : schéma simple, sans recyclage de l'énantiomère S, et sans racémisation.

- Sur la figure 7: schéma avec recyclage de l'énantiomère S, et avec racémisation.

Les schémas de blocs d'opération peuvent aussi bien se lire en flux (kg/h) qu'en quantité (kg) traitée par opération.

2.2 Définition des relations entre les entrées et les sorties des blocs d'opération

Les données du brevet US 6 444 854 B1 et quelques hypothèses raisonnables, nous permettent de décrire le comportement de chaque opération des figures 6 et 7. Chaque opération est décrite par son effet sur les flux (ou les quantités).

Le schéma de blocs d'opération de la figure 6 ne contient que deux blocs d'opération :

- un bloc d'opération de mélange *EluMix*
- un bloc d'opération de séparation-evaporation *SMB_Evap* qui permet de séparer les énantiomères et de les concentrer.

Le schéma de blocs d'opération de la figure 7 contient les opérations de la figure 6 et en plus :

- un autre bloc d'opération de mélange *RacMix*

- un bloc d'opération de réaction racémisation *Racemization*: il convertit l'énantiomère-S en énantiomère-R. La transformation s'arrête lorsqu'un mélange racémique est obtenu
- un bloc d'opération de *PrecipFiltr*
- un bloc d'opération de séchage du racémate *RacDry*

Nous allons désormais détailler le fonctionnement de ces différentes opérations en termes des relations entre les entrées et les sorties des blocs d'opération.

Les mélangeurs *EluMix* et *RacMix* reçoivent les matériaux d'entrée à 25°C.

Les compositions et température de sorties sont une simple combinaison linéaire de celles d'entrées ; les mélanges sont ici des liquides supposés idéaux.

Pour les opérations de séparation, les ratios de séparation décrivent comment un composé contenu dans les flux d'entrée est réparti entre les flux de sortie. La température, l'état, les rapports de fractionnement sont donnés (possiblement devinés/intuités/souhaités) par l'utilisateur. Ils sont soit de 0% ou 100% dans les tableaux ci-dessous, mais peuvent prendre n'importe quelle valeur comprise entre 0% et 100% (voir Table 33). Les valeurs sont basées sur l'expérience ou l'intuition de chimiste ou par quelques résultats du brevet US 6 444 854 B1.

- le bloc d'opération *PrecipFiltr* convertit Na+ et CI- (dissous) en NaCl solide qui est envoyé à *SaltsOut.* (voir Table 30) :

Table 30: ratios de séparation de l'opération *PrecipFiltr*

|  | SaltsOut | S2 |
|---|---|---|
| NaCl (Solide) | 100% | 0% |
| Autres espèces | 0% | 100% |

- le bloc d'opération *RacDry* est décrit avec les paramètres de la Table 31. Ils décrivent une opération de séchage du racémate parfaite. Pour cette séparation, l'ensemble de la charge est porté à 85°C avec évaporation de *SolvOut* (enthaplie de changement d'état de 850 kJ/kq) puis les deux sorties sont ramenées à 25°C à l'état liquide.

Table 31: ratios de séparation du bloc d'opération *RacDry*

|  | SolvOut | Rac_Loop |
|---|---|---|
| R-Sertraline-tétralone | 0% | 100% |
| S-Sertraline-tétralone | 0% | 100% |
| Autres espèces | 100% | 0% |

La performance de la séparation LMS peut être calculée à partir des résultats expérimentaux exposés dans le brevet. Nous utilisons les données de la troisième expérience (voir Table 32).

Table 32: Performances LMS rapportées au cours de la troisième expérience (brevet US 6 444 854).

| Pureté de l'énantiomère le moins retenu (%) | 99.7 |
|---|---|
| Rendement de récupération de l'énantiomère le moins retenu (%) | 98,4 |
| Volume calculé d'éluent nécessaire (L/g d'énantiomères) | 0,40 |

À partir de cette information, nous pouvons calculer les ratios de séparation de l'opération *SMB_evap* (voir Table 33). Nous considérons que l'opération *SMB_evap* comprend le processus chromatographique, mais aussi d'évaporation pour enlever une grande partie des solvants. Ces solvants évaporés sont recyclés pour raisons d'économie. Pour ce faire, toute la charge de l'opération est portée à 85°C, ce qui vaporise le flux *Solv* (enthalpie de changement d'état de 850 kJ/kg). Ensuite, les trois flux de sortie sont ramenés à 25°C à l'état liquide.

Table 33: ratios de séparation du bloc d'opération *SMB_evap* dans le cas de base

|  | Raffinat | Extrait | Solvant |
|---|---|---|---|
| R-Sertraline-tétralone | 98,4% | 1,6% | 0% |

(suite)

|  | Raffinat | Extrait | Solvant |
|---|---|---|---|
| S-Sertraline-tétralone | 0,12% | 99,88% | 0% |
| Méthanol | 0,5% | 0,5% | 99% |
| Acétonitrile | 0,5% | 0,5% | 99% |

2.3 Détermination de bilans globaux

Les flux d'entrée sont dimensionnés pour obtenir un débit de 12,5 kg/h (soit 100 tonnes par an pour 8 000 heures) d'énantiomère-R dans le flux *Raffinate.*

Le débit du flux *FreshEluent* est calculé de façon à ce que le flux S3 satisfasse aux conditions de la table 3.

Dans le cas sans racémisation, nous obtenons les débits d'entrée exposés dans la Table 34. La puissance thermique à apporter au procédé s'élève à 1042 kW, la puissance à retirer est de même valeur.

Table 34: description des flux sortants intrants, et internes du procédé dans le cas sans recyclage de l'énantiomère S, et sans racémisation.

|  | Flux | Matières premières | Débit / Quantités |
|---|---|---|---|
| SORTANTS | *Raffinate (contenant R-énantiomère)* |  | 32.58 kg/h (dont 12,5 kg/h de R) |
|  | *Extract* |  | 32.96 kg/h |
| INTRANTS | *FreshRacemate* | mélange racémique d'énantiomères | 25,41 kg/h |
|  | *FreshEluent* | 90/10 mélange acétonitrile -méthanol | 50,8 L/h |
| INTERNES | *S3* |  | 4013 kg/h |
|  | *Solvent* |  | 3973 kg/h |

En cas de recyclage et de racémisation de l'énantiomère S (voir voir figure 7), le flux (quantité) *FreshRacemate* est largement abaissé (voir Table 35).

Les quantités de matières premières nécessaires à l'opération *Racemization* (C2H3N_feed1, NaOH_feed, MeOH_feed, MeOH_feed2, C2H3N_feed2, HCl_feed) et les débits sont calculés sur la base de l'expérience de racémisation décrite dans le Brevet US 6 444 854 pour garder les proportions entre ces matières premières et la quantité d'énantiomères à racémiser. En particulier, le débit (quantité) d'acétonitrile à fournir est égal à 30 litres pour chaque kilogramme de S-énantiomère qui est alimenté à la racémisation. Le flux (quantité) *Extr* qui arrive à l'opération *Racemization* contient de l'acétonitrile qui est déduit de l'alimentation C2H3N_feed1.

Les paramètres de performance de la chromatographie en LMS (voir Table 33) ne sont pas affectés par la racémisation.

Dans ce deuxième cas, la puissance thermique à apporter au procédé s'élève à 1182 kW ; la puissance thermique à retirer s'élève à 1131 kW.

Table 35: description des intrants, sortants et internes du procédé dans le cas avec recyclage de l'énantiomère S et racémisation.

|  | Flux | Matières premières | Débit/quantités |
|---|---|---|---|
| SORTANTS | *Raffinate (contenant R-énantiomère)* |  | 32,65 kg/h (dont 12,5 kg/h de R) |
|  | *Solv_Out* |  | 323,1 kg/h |
|  | *Salt_Out* |  | 0,15 kg/h |

(suite)

| | Flux | Matières premières | Débit/quantités |
|---|---|---|---|
| INTRANTS | *FreshRacemate* | mélange racémique d'énantiomères | 12,52 kg/h |
| | *FreshEluent* | 90/10 mélange acétonitrile -méthanol | 51 L/h |
| | *C2H3M_feed* | acétonitrile | 106,88 L/h |
| | *MaOH_feed* | NaOH | 0,1 kg/h |
| | *MeOH_feed* | méthanol | 0,07 L/h |
| | *MeOH_feed2* | méthanol | 12,89 kg/h |
| | *C2H3N_feed 2* | acétonitrile | 257,76 L/h |
| | *HCl_feed* | HCl | 1,94 kg/h |
| INTERNES | *S3* | | 4029 kg/h |
| | *S5* | | 25,42 kg/h |
| | *Extr* | | 33,04 kg/h |
| | *Solvent* | | 3989 kg/h |
| | *S1* | | 117,6 kg/h |
| | *S2* | | 336,0 kg/h |
| | *RacLoop* | | 12,90 kg/h |

Il convient de noter que les résultats de la Table 34 et de la Table 35 sont obtenus par prise en compte des relations entre les entrées et les sorties de chaque bloc d'opération et des connections entre blocs d'opération et qu'il s'agit d'un premier résultat de calcul de simulation.

Le système peut maintenant utiliser le résultat de la simulation (c'est-à-dire les flux ou quantités circulant dans les procédés donnés dans les tables 34 et 35) pour déterminer une première évaluation des coûts de productions variables. Pour l'évaluation économique, nous considérons les hypothèses suivantes qui proviennent de l'expérience. Ce sont des ordres de grandeur ;

- les prix des matières premières sont ceux énumérés dans la Table 36
- les sorties de solvants sont considérées comme déchets avec un coût de traitement de 0,15 €. Le coût du traitement des autres extrants secondaires (*Extr* pour le processus sans racémisation, *SaltsOut* pour le processus avec racémisation) est négligé.
- pour les échanges thermiques, on considère un coût de 0.10 €/kWh lorsqu'il s'agit d'énergie à apporter au procédé (chauffage/évaporation) et de 0.05 €/kWh lorsqu'il faut retirer cette énergie (refroidissement/condensation).

Table 36 : prix des matières premières

| Matières premières | Coût (€/kg) |
|---|---|
| Sertraline-tétralone racémate | 100 |
| Acétonitrile | 20 |
| Méthanol | 2 |
| 90/10 mélange acétonitrile-méthanol | 19 |
| NaOH | 0,43 |
| HCl | 0,75 |

Les informations contenues dans les tables 34 à 36 conduisent aux coûts variables des deux versions du procédé exposés dans la Table 37.

Table 37: contribution de chaque entrée à la partie variable du coût de production d'énantiomère-R. Les valeurs sont exprimées en €/kg d'énantiomère-R dans le flux *Raff*.

| | Procédé 1 | Procédé 2 | Procédé 3 |
|---|---|---|---|
| | Sans racémisation | Avec recyclage de l'énantiomère-S et racémisation | Avec recyclage de l'énantiomère-S, racémisation et recyclage de l'acétonitrile |
| FreshRacemate | 203,25 | 100,12 | 100,12 |
| Total Acétonitrile (deux alimentations) | - | 461,10 | - |
| Total Méthanol (deux alimentations) | - | 2,07 | 2,07 |
| Frais éluant | 60,99 | 61,27 | 61,27 |
| NaOH_feed | - | < 0,01 | < 0,01 |
| HCl_feed | - | 0,12 | 0,12 |
| Traitement des effluents | 0,00 | 3,88 | 0,46 |
| Énergie | 12,51 | 13,58 | 15,68 |
| Total (€/kg de R dans Raffinate) | 276,75 | 642,14 | 190,53 |

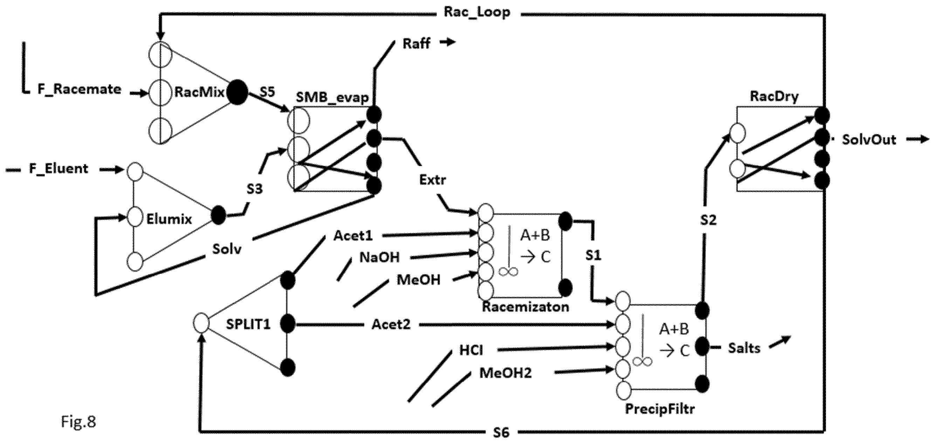

Nous pouvons remarquer que le recyclage et la racémisation (procédé 2) sont fortement pénalisés par la consommation d'acétonitrile qui est utilisé comme solvant de réaction. Une purification et un recyclage partiel de cet acétonitrile (voir figure 8) pourrait être considéré. Nous utilisons la méthodologie précédente pour évaluer cette troisième option. On montre qu'avec un recyclage de 94% de l'acétonitrile, aucune addition d'acétonitrile n'est nécessaire, mais que le coût de l'énergie est augmenté de 2,10 € par kg d'énantiomère-R dans *Raff*. Le coût variable total est égal à 190,53 €/kg, ce qui est plus intéressant que les autres options.

Avec cette première approche, nous avons montré que l'option 2 est très susceptible d'être peu intéressante d'un point de vue économique et en termes de production de déchets. Dans la suite, nous ne considérerons que les options 1 et 3.

Etape 3 : Schéma d'équipements

3.1 Transformation en schéma d'équipements

Dans l'étape 3.1, chacun des blocs d'opération des schémas précédents est affecté à un équipement.

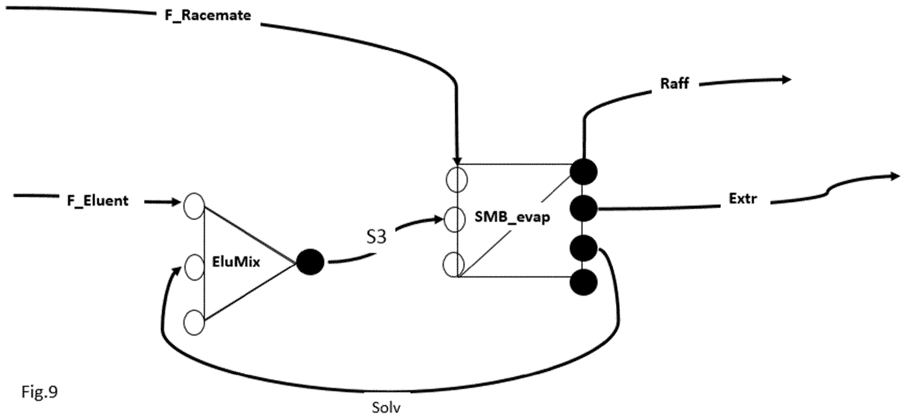

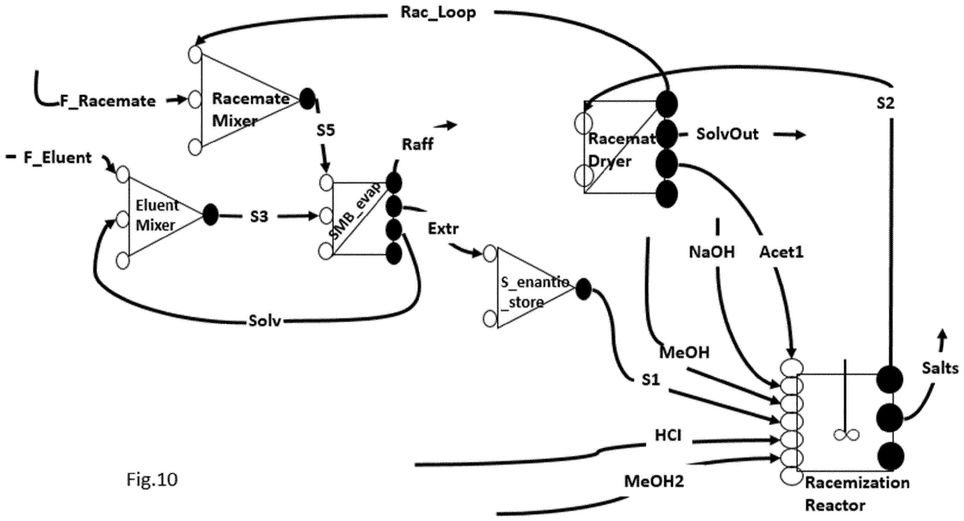

La Table 38 fournit une correspondance entre les blocs d'opération et les équipements. Les diagrammes d'équipement générés sont illustrés sur les figures 9 et 10. Les équipements utilisés ici appartiennent tous à la bibliothèque BEQGEN.

**Table 38 : correspondance entre blocs d'opération et équipements. Les types d'équipements correspondent à ceux définis dans la bibliothèque BEQGEN.**

| Bloc d'opération | équipement (nom) | équipement (type) | Volume (m³) |
|---|---|---|---|
| *RacMix* | *RacemateMixer* | Mélangeur | 2.5 |
| *EluMix* | *EluentMixer* | Mélangeur | 4.6 |
| *Racemisation* | *RacemisationReactor* | Réacteur | 6 |
| *PrecipFiltr* | | | |
| *Split1* | | | |

(suite)

| Bloc d'opération | équipement (nom) | équipement (type) | Volume (m³) |
|---|---|---|---|
| *RacDry* | *RacemateOryer* | Séparateur | 6 |
| *SMB_evap* | *SMB_evap* | Séparateur | 0.3 |

Pour cet exemple, une opération est affectée à un équipement (Table 38), hormis pour *RacemizationReactor* qui regroupe plusieurs opérations.

### 3.2 Définition des opérations effectuées sur chaque procédé

Dans le cas du diagramme de la figure 9, les deux équipements fonctionnent en continu avec les alimentations exposés dans la Table 34.

Dans le cas du diagramme de la figure 10, une partie du procédé est opérée en continu (*EluentMixer* et *SMB_evap*) là où une autre partie du procédé fonctionne de façon discontinue (*RacemizationReactor* et *RacemateDryer*) sur des cycles de 20 heures (dont 10 heures de réaction). Ces cycles suivent une procédure de la même nature que celle exposée dans la Table 22 et la Table 23 de l'exemple précédent. La procédure de ce second cas d'étude ne sera pas détaillée ici.

Les équipements *RacemateMixer* et *S_enantio_store* doivent donc être en mesure de stocker la production d'un cycle. Ce second équipement de stockage est créé par nécessité pratique, il n'a pas d'équivalent dans le diagramme d'opérations car l'approche utilisée a permis de faire jusqu'ici abstraction des contraintes d'interfaçage continu-discontinu.

### 3.3 Détermination par le système des paramètres de fonctionnement

Les opérations effectuées dans les équipements des deux procédés étudiés sont décrites suivant des modèles de la bibliothèque MEQ1. Ces modèles suivent les mêmes principes directeurs que ceux de la bibliothèque MOP1 (pas d'utilisation de données physico-chimiques, description macroscopique des phénomènes) ainsi que la même structure. De ce fait, les ratios de partition des tables 30 à 33, de même que la description de la réaction de racémisation, conservent toute leur validité. Les bilans de matières et d'énergies entrantes et sortantes sont donc inchangés (voir Table 34 pour le cas sans racémisation).

Contrairement aux diagrammes d'opérations, les diagrammes d'équipements gèrent les notions de temps et de taille d'équipements. Ainsi, on peut accéder à des paramètres de fonctionnement tels que le taux maximal de remplissage ou la productivité de tel ou tel équipement. Les valeurs de ces paramètres de fonctionnement permettent à l'utilisateur de juger de la pertinence pratique des tailles d'équipements et temps d'opérations qu'il a spécifié. Dans le cas présent, compte-tenu des tailles initiales données dans la Table 38, le taux d'occupation maximal du réacteur de racémisation et du sécheur de racémate est d'environ 130%, ce qui évidemment irréaliste. De même, on aboutirait pour le *SMB_evap* à une productivité de 42 kg d'énantiomère R par heure et par m³, là où les valeurs courantes pour de telles séparations sont plutôt de l'ordre de 25 kg d'énantiomère R par heure et par m³. Pour ce qui est de la racémisation, le document US 6,444,854 nous rapporte une expérience où 100 g d'énantiomère S sont traités en 6 heures dans un volume de 3 à 5 litres de solution. Cette réaction, telle qu'elle est menée dans le cadre de cette expérience possède donc une productivité de 3 - 4 $kg_{s,\ entrée}/(h.m^3_{contenant})$.

Un processus itératif permet d'ajuster les tailles des équipements de façon à ce que les paramètres de fonctionnement résultants atteignent des valeurs conformes aux attentes. On aboutit ainsi aux tailles et paramètres de fonctionnement exposés dans la Table 39. On constate notamment que, pour le réacteur de racémisation, la contrainte sur le taux de remplissage impose de travailler à une productivité inférieure à celle autorisée par les phénomènes chimiques.

**Table 39** : dimension des principaux équipements pour une production de 12,5 kg/h d'énantiomère R ; l'éventuelle racémisation étant effectuée par cycles de 20 heures

| Equipement | Vol. (m³) | Paramètre | Valeur |
|---|---|---|---|
| *SMB_evap* | 0.5 | Productivité | 25 $kg_R/(h.m^3)$ |
| *RacemateDryer* | 10 | Remplissage | environ 80% |
| *RacemizationReactor* | 10 | Remplissage | environ 80% |
|  |  | Productivité | 2,5 $kg_{S,\ entrée}/(h.m^3_{contenant})$ |

(suite)

| Equipement | Vol. (m³) | Paramètre | Valeur |
|---|---|---|---|
| *RacemateMixer* | | | |
| *EluentMixer* | Non considérés | | |
| *S_enantio_store* | | | |

Nous disposons à ce stade du dimensionnement de tous les équipements essentiels à la production.

Le système peut se servir de cette information pour effectuer une première estimation économique des coûts fixes.

Les coûts des matières premières sont ceux de Table 36. Les hypothèses sur les coûts variables restent les mêmes que précédemment.

Le mode de calcul du coût des équipements est exactement le même que dans l'exemple 1. Avec les informations données dans la Table 40 on peut donc estimer le coût des différents équipements.

Table 40 : taille, paramètres économiques et coût de chaque équipement ;

| équipement | Taille | Unité de taille | Taille de réf. | Prix de réf. (k€) | Élasticité | Coût (k€) de l'équipement |
|---|---|---|---|---|---|---|
| *SMB_evap* | 0,5 | m3 | 1 | 30 000 | | 19 793 |
| *RacemizationReactor* | 10 | m3 | 3 | 1 000 | 0,6 | 2 060 |
| *RacemateDryer* | 10 | m3 | 3 | 1 500 | | 3 090 |
| Total CAPEX (sans racémisation ni recyclage d'acétonitrile) | | | | | | 19 793 |
| Total CAPEX (avec racémisation et recyclage d'acétonitrile) | | | | | | 24 940 |

Comme dans l'exemple 1, la contribution de l'équipement au coût de production est calculée en considérant que le coût d'investissement de l'équipement est amorti sur 64 000 heures d'utilisation. Nous considérons également un coût d'entretien qui équivaut à 5% de CAPEX par an (soit 8,000 heures).

Dans le cas présent, le coût du travail est considéré comme secondaire (pour le but d'illustration) mais pourrait être considéré. Nous considérons un coût de contrôle de la qualité de 100 000 €/an et un coût frais généraux qui équivaut 25% des autres coûts fixes. En ce qui concerne le coût de l'adsorbant utilisé pour la chromatographie en LMS, nous considérons un coût d'achat de 10 000 €/kg, une durée d'utilisation de 16 000 heures et une masse de 1 kg par kg d'énantiomère-R dans *Raff* par jour. Pour le cas impliquant la racémisation, on considère que le procédé est arrêté 10 heures entre les cycles de 20 heures.

Table 41: coût total de production (en € par kg d'énantiomère-R dans *Raff*) avec ventilation des coûts fixes

| | Option 1 | Option 3 |
|---|---|---|
| | Sans racémisation | Avec racémisation de l'énantiomère-S et recylage de l'acétonitrile |
| Amortissement de l'équipement | 24,74 | 46.79 |
| Entretien | 9,89 | 18.72 |
| Adsorbant | 15,00 | 15,00 |
| Contrôle de la qualité | 1,00 | 1,50 |
| Frais Généraux | 12,66 | 20.50 |
| Total des coûts fixes | 63,29 | 102.51 |
| Coûts variables totaux | 276.75 | 190.53 |
| Total (€/kg de R dans Raffinate) | 340.04 | 293.04 |

En raison de la présence d'équipements additionnels, l'option 3 a des coûts fixes plus élevés que l'option 1. Toutefois, en cumulant des coûts variables et fixes, l'option 3 semble plus attractive. Dans les études suivantes, seule cette configuration sera envisagée.

Les calculs préliminaires présentés précédemment ont soulevé l'idée d'une configuration de procédé incluant la racémisation de l'énantiomère-S et le recyclage du solvant de racémisation. Ces calculs ont également indiqué que, pour l'échelle de production envisagée, cette configuration était la plus intéressante, ce qui permet d'exclure les deux autres.

L'approche proposée dans l'invention permet de tester l'impact de certains paramètres macroscopiques de performance pour déterminer ceux qui ont l'impact le plus élevé sur le coût de production. Dans le cas présent, cette étude est assez simple. En effet, la Table 39 nous indique que, même avec des phénomènes chimiques un peu plus lents ou beaucoup plus rapides qu'estimé, la taille du réacteur de racémisation ne serait pas modifiée puisque celle-ci est fixée par la contrainte du taux de remplissage. Cela n'aurait donc aucun impact sur les coûts.

A l'inverse, la performance du SMB (« Simulated Moving Bed », acronyme anglais pour « Lit Mobile Simulé ») a un impact direct sur les coûts via la consommation d'éluent, la quantité d'adsorbant ou encore la taille du SMB qui elle est directement dépendante de la productivité.

Nous pouvons immédiatement voir que les performances de *SMB_evap* a un impact beaucoup plus grand que celui de la racémisation. Il s'ensuit que, pour avoir une meilleure compréhension du processus, nous devons axer nos études sur le SMB plutôt que sur la racémisation.

3.4 Détermination par le système de paramètres de fonctionnement suivant un modèle standard (MEQ2) ou détaillé (MEQ3)

En utilisant des schémas et des modèles très simples, nous avons pu simuler différentes options de procédé et converger vers une option raisonnable sans connaissance physico-chimique particulière. Nous avons identifié que le *SMB_evap* est un équipement particulièrement important au niveau économique. Si le projet a de l'intérêt, il est évident que l'on ne peut pas se contenter du niveau Guess mais que des modèles mécanistiques/prédictifs doivent être utilisés pour confirmer, préciser, voire infirmer les hypothèses du niveau Guess. Par contre, effectuer à ce stade une autre étude de la racémisation serait une dépense inutile de temps et d'argent.

Pour une étude plus avancée du *SMB_Evap,* l'utilisation de modèles mécanistiques (bibliothèque MEQ2) qui nécessitent la détermination de paramètres physico-chimiques est nécessaire. Le cas échéant, il pourrait même se révéler nécessaire d'utiliser des modèles de la bibliothèque MEQ3 qui sont capables de simuler finement le comportement d'un équipement donné. Concernant le fonctionnement des LMS, un modèle de type MC-LDF serait certainement à recommander (Chromatographic Processes: modeling, simulation and design, Roger-Marc Nicoud, Cambridge University Press, 2015*).*

L'approche que nous proposons nous a permis de choisir une bonne configuration sans exiger la mesure des paramètres physico-chimiques. Maintenant, pour aller plus loin, nous avons besoin de ces paramètres physico-chimiques, mais nous savons précisément lesquels et pourquoi nous devons les mesurer.

L'approche descendante de l'invention commence par des calculs modérément complexes et économes en informations. Grâce aux résultats de ces premiers calculs, nous savons quels raffinements ont priorité, quelles informations supplémentaires doivent être

L'effet technique de la méthode et du dispositif suivant l'invention est de permettre l'obtention d'un schéma d'équipements d'une installation industrielle, puis la réalisation et l'exploitation de celle-ci. Grâce à l'invention, ce schéma d'équipements peut être obtenu plus rapidement que dans les méthodes traditionnelles. En outre, grâce aux options d'itération, avec amélioration des éléments les plus critiques, le schéma d'équipement obtenu peut présenter des paramètres de fonctionnement meilleurs que ceux obtenus par les méthodes traditionnelles. La méthode et le dispositif de l'invention peuvent être utilisées par un chimiste de laboratoire, qui n'a pas nécessairement les compétences et l'expérience d'un ingénieur de procédé.

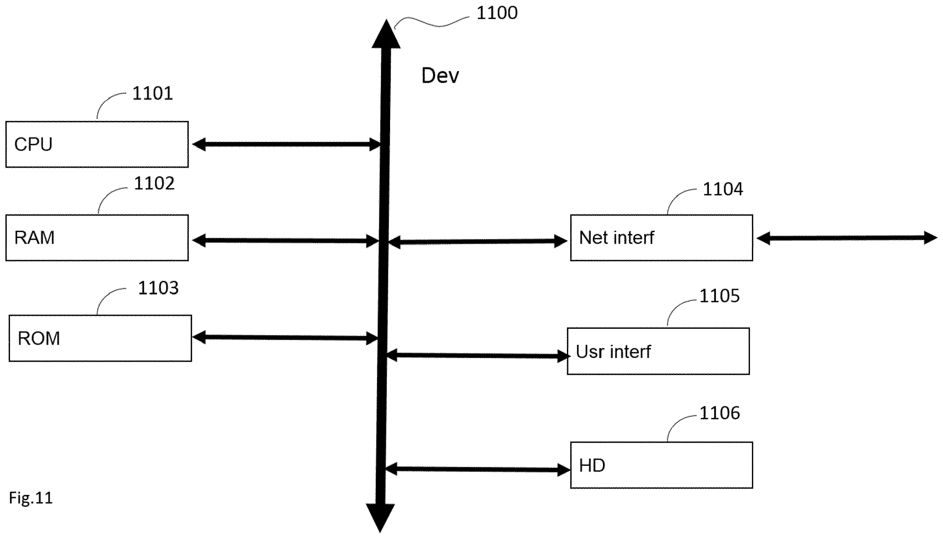

**Figure 11** est un diagramme bloc représentant un dispositif **1100** pour implémenter un ou plusieurs modes de réalisation de l'invention. Les diverses parties du système décrites ci-dessus peuvent avoir une même structure que le dispositif **1100.**

Le dispositif **1100** comprend un bus de communication relié à :

- une unité centrale de traitement **1101** tel qu'un microprocesseur, autrement nommée CPU ;
- Une mémoire à accès aléatoire **1102,** autrement nommée RAM, pour stocker un code exécutable du procédé des modes de réalisation de l'invention ainsi que les registres adaptés pour enregistrer les variables et les paramètres nécessaires à la mise en oeuvre du procédé conformément aux modes de réalisation, la capacité de la mémoire pouvant être augmentée par une RAM optionnelle connectée par exemple à un port d'expansion ;
- Une mémoire à lecture seule **1103,** autrement nommée ROM, pour stocker les programmes d'ordinateur utilisés pour mettre en oeuvre les modes de réalisation de l'invention ;

- une interface de réseau **1104,** qui est typiquement connectée à un réseau de communication sur lequel des données numériques devant être traitées sont transmises ou reçues. L'interface de réseau **1104** peut être une seule interface de réseau ou être composée d'un ensemble d'interfaces de réseau différentes (par exemple des interfaces filaires et sans-fil, ou différentes sortes d'interfaces filaires ou sans-fil). Les données sont écrites sur l'interface de réseau pour transmission ou lues à partir de l'interface réseau pour réception sous le contrôle de l'application logicielle fonctionnant dans le CPU **1101** ;
- Une interface utilisateur **1105** pour la réception des saisies d'un utilisateur ou pour l'affichage d'information à l'utilisateur;
- Un disque dur **1106** autrement nommé HD
- un module entrée/sortie **1107** (autrement nommé I/O) pour envoyer/recevoir des données de/vers des dispositifs tels qu'une source vidéo ou un écran d'affichage

Le code exécutable peut être stocké, soit dans la mémoire à lecture seule 11**03**, soit sur le disque dur **1106,** ou sur un support numérique amovible tel qu'un disque par exemple. Selon une variante, le code exécutable des programmes peut être reçu au moyen d'un réseau de communication, via l'interface de réseau 11**04**, afin d'être stocké sur un des supports de stockage du dispositif de communication **1100,** tel que le disque dur **1106,** avant d'être exécuté.

L'unité centrale de traitement **1101** est adaptée pour contrôler et diriger l'exécution des instructions ou des portions de code logiciel du programme des programmes conformément aux modes de réalisation de l'invention, lesquelles instructions sont stockées sur l'un des supports de stockage précités. Après avoir été mise en service, la CPU **1101** est capable d'exécuter les instructions à partir de la mémoire RAM principale **1102** en rapport avec une application logicielle par exemple après que ces instructions ont été chargées à partir du programme ROM **1103** ou sur le disque dur (HD) **1106.** Cette application logicielle, lorsqu'elle est exécutée par la CPU **1101,** amène les étapes de procédé à être mises en oeuvre conformément aux modes de réalisation.

Les dessins et la description détaillée qui leur est associée sont fournis à titre d'exemple. Bien que l'invention soit illustrée par ces dessins et que des modes de réalisation soient décrits dans les détails, l'invention n'est en aucun cas limitée aux seuls modes de réalisation divulgués dans les présentes. D'autres variantes de l'invention divulguée peuvent être extrapolées et réalisées par les hommes de métier souhaitant mettre en oeuvre l'invention revendiquée à partir d'une étude des dessins, de la divulgation et des revendications en annexe.

Dans les revendications, le mot « comprenant » n'exclut pas autres éléments ou étapes, et l'article indéfini singulier, « un » ou « une » n'exclut pas une pluralité. Le simple fait que différentes caractéristiques soient citées simultanément dans des revendications différentes dépendantes n'indique pas qu'une combinaison de ces caractéristiques ne peut pas être utilisée de façon avantageuse. Un quelconque signe de référence dans les revendications ne peut être interprété comme une limitation de la portée de l'invention.

## Revendications

1. Méthode mise en oeuvre par un ordinateur de simulation d'une installation industrielle pour l'exploitation d'un procédé chimique ou biochimique de production d'une ou plusieurs substances finales, respectant des contraintes relatives à des paramètres de fonctionnement de l'installation industrielle, comprenant la productivité, le rendement, des paramètres de qualités des produits tels que la pureté, la production de déchets, la consommation d'énergie, les coûts d'investissement ou d'exploitation, dans laquelle on dispose

   • d'une bibliothèque BBOP de blocs d'opération BOP, chaque bloc d'opération BOP représentant une opération élémentaire d'un procédé chimique ou biochimique par ses entrées et ses sorties,
   • d'une bibliothèque d'équipements génériques BEQGEN et d'une bibliothèque d'équipements spécifiques BEQSPEC, lesdits équipements étant aptes à exécuter des opérations des dits blocs d'opération,

   comprenant :

   à l'étape 1,

      1.1. la définition par l'utilisateur des matières premières entrant dans le procédé ;
      1.2. la décomposition de ces matières premières en espèces chimiques ;
      1.3. la fourniture par l'utilisateur de l'information des espèces chimiques ou des matières biologiques produites dans le procédé ;

à l'étape 2,

2.1 la définition d'un schéma de blocs d'opération représentatif du procédé chimique ou biochimique par agencement de blocs d'opération BOP sélectionnés dans la bibliothèque BBOP de blocs d'opération ;

2.2 l'ajout au schéma de blocs d'opération d'une définition des relations entre les entrées et les sorties de chacun des blocs d'opération BOP sélectionnés ;

2.3 la détermination par l'ordinateur, sur base des données obtenues aux étapes 1.1 à 2.2, de bilans globaux du procédé ces bilans globaux concernant un ou plusieurs paramètres sélectionnés parmi la production de la une ou plusieurs substances finales, la consommation de matières premières ou d'énergie ;

2.4 optionnellement, l'itération des étapes 2.1, et ou 2.2 puis 2.3, jusqu'à obtention de bilans globaux améliorés ;

à l'étape 3,

3.1. la transformation du schéma de blocs d'opération en un schéma d'équipements représentant ladite installation industrielle, dans laquelle des équipements sont choisis dans la bibliothèque d'équipements génériques BEQGEN ou dans la bibliothèque d'équipements spécifiques BEQSPEC pour réaliser les opérations d'un ou plusieurs blocs d'opération BOP du schéma de blocs d'opération, jusqu'à la transformation de tous les blocs d'opération BOP du schéma de blocs d'opération ;

3.2. la définition des caractéristiques des différents équipements génériques ou spécifiques du schéma d'équipement, ces caractéristiques comprenant des données de productivité, de volume, de temps de réaction, de vidange ou de chauffage, ou de coûts d'investissement ou d'exploitation ;

3.3. la détermination par l'ordinateur, sur base des données obtenues aux étapes 3.1 à 3.2, des dits paramètres de fonctionnement de l'installation industrielle ;

3.4. optionnellement, itération des étapes 3.1, et ou 3.2 puis 3.3, jusqu'à obtention paramètres de fonctionnement améliorés.

2. Méthode suivant la revendication 1 **caractérisée en ce qu'**on dispose en outre d'une première bibliothèque de modèles d'opération MOP1 définissant les relations entre les entrées et les sorties des dits blocs d'opération BOP, et qu'à l'étape 2.2, la définition des relations entre les entrées et les sorties d'au moins un des blocs d'opération BOP sélectionnés est extraite de ladite première bibliothèque, complétée optionnellement par la fourniture de paramètres de ce modèle par l'utilisateur.

3. Méthode suivant l'une quelconque des revendications précédentes **caractérisée en ce qu'**elle comprend, en outre, à l'issue de l'étape 2.3 ou 2.4, une étape 2.5 dans laquelle on remplace les relations entre les entrées et les sorties de certains des blocs d'opération BOP par des relations extraites d'une seconde bibliothèque de modèles d'opération MOP2, cette seconde bibliothèque de modèles d'opération MOP2 comportant des caractéristiques plus élaborées, sélectionnées parmi les informations thermodynamiques, ou de composition de phases, et on détermine les bilans de procédé, suivant l'étape 2.3, sur base de ces caractéristiques améliorées.

4. Méthode suivant l'une quelconque des revendications précédentes **caractérisée en ce qu'**on dispose d'une première bibliothèque de modèles d'équipements MEQ1 comportant des caractéristiques de performances des équipements, et **en ce qu'**à l'étape 3.2, la définition des caractéristiques d'au moins un des équipements génériques ou spécifiques du schéma d'équipement est extraite de la ladite première bibliothèque de modèles d'équipements MEQ1, complétée optionnellement par la fourniture de paramètres de ce modèle par l'utilisateur.

5. Méthode suivant l'une quelconque des revendications précédentes **caractérisée en ce qu'**elle comprend, en outre, à l'issue de l'étape 3.3 ou 3.4, une étape 3.5 dans laquelle on remplace les caractéristiques de certains des équipements génériques ou spécifiques du schéma d'équipement par des caractéristiques extraites d'une seconde bibliothèque de modèles d'équipements MEQ2, les modèles issus de cette seconde bibliothèque étant plus élaborés et permettant notamment de calculer des conversions dans des réacteurs ou des performances de séparation en distillation à partir de données physico-chimiques, et on détermine les paramètres de fonctionnement de l'installation industrielle, suivant l'étape 3.3, sur base de ces caractéristiques améliorées.

6. Méthode suivant les revendications 4 ou 5 **caractérisée en ce qu'**elle comprend, en outre, à l'issue de l'étape 3.3 ou 3.4, une étape 3.5 dans laquelle on remplace les caractéristiques de certains des différents équipements génériques ou spécifiques du schéma d'équipement par des caractéristiques extraites d'une troisième bibliothèque de modèles d'équipements MEQ3, les modèles issus de cette troisième bibliothèque étant des modèles détaillés et on détermine les paramètres de fonctionnement de l'installation industrielle, suivant l'étape 3.3, sur base des caractéristiques améliorées.

7. Méthode suivant l'une quelconque des revendications précédentes **caractérisée en ce qu'**elle comprend, en outre, la réalisation de l'installation industrielle conçue et/ou l'exploitation du procédé chimique ou biochimique dans cette installation.

8. Dispositif pour la simulation d'une installation industrielle pour l'exploitation d'un procédé chimique ou biochimique de production d'une ou plusieurs substances finales, respectant des contraintes relatives à des paramètres de fonctionnement de l'installation industrielle, comprenant la productivité, le rendement, des paramètres de qualités des produits tels que la pureté, la production de déchets, la consommation d'énergie, les coûts d'investissement ou d'exploitation, **caractérisé en ce qu'**il comprend des moyens de mise en oeuvre d'une méthode suivant l'une quelconque des revendications 1 à 7.

9. Dispositif suivant la revendication 8 comprenant au moins un ordinateur, une mémoire dans laquelle sont mémorisées

   • une bibliothèque BBOP de blocs d'opération BOP, chaque bloc d'opération BOP représentant une opération élémentaire d'un procédé chimique ou biochimique par ses entrées et ses sorties,
   • une première bibliothèque de modèles d'opération MOP1 définissant les relations entre les entrées et les sorties des dits blocs d'opération BOP,
   • une bibliothèque d'équipements génériques BEQGEN et d'une bibliothèque d'équipements spécifiques BEQSPEC, lesdits équipements étant aptes à exécuter des opérations des dits blocs d'opération,
   • une première bibliothèque de modèles d'équipements MEQ1 comportant des caractéristiques de performances desdits équipements ;

   des moyens d'entrée/sortie adaptés pour permettre à un utilisateur de fournir les données requises, et de prendre connaissances des bilans globaux et paramètres de fonctionnement déterminés dans une méthode suivant l'une quelconque des revendications 1 à 7 ;
   des moyens de calcul pour déterminer les bilans globaux et paramètres de fonctionnement déterminés dans ladite méthode.


**Patentansprüche**

1. Methode zur Umsetzung durch einen Rechner einer Simulation einer Industrieanlage zur Nutzung eines chemischen oder biochemischen Verfahrens zur Produktion einer oder mehrerer finaler Substanzen bei Einhaltung der Anforderungen, die sich auf Betriebsparameter der Industrieanlage beziehen, umfassend die Produktivität, die Leistung, Qualitätsparameter der Produkte wie Reinheit, Abfallerzeugung, Energieverbrauch, die Investitions- oder Betriebskosten, wobei man verfügt über

   • eine BBOP-Bibliothek aus BOP-Operation-Blocks, wobei jeder BOP-Operation-Block eine elementare Operation eines chemischen oder biochemischen Verfahrens durch seine Eingänge und seine Ausgänge repräsentiert,
   • eine BEQGEN-Bibliothek generischer Ausrüstungen und eine BEQSPEC-Bibliothek spezifischer Ausrüstungen, wobei die Ausrüstungen imstande sind, Operationen der Operation-Blocks auszuführen,

   umfassend:

   in Schritt 1,

      1.1 die Definition der in das Verfahren eingehenden Rohstoffe durch den Benutzer;
      1.2 die Zerlegung dieser Rohstoffe in chemische Substanzen,
      1.3 die Bereitstellung der Information über die im Verfahren hergestellten chemischen Substanzen oder biologischen Materialien durch den Benutzer;

   in Schritt 2,

      2.1 die Definition eines Operation-Blocks-Schemas, das für das chemische oder biochemische Verfahren repräsentativ ist, durch Anordnung von in der BBOP-Operation-Blocks-Bibliothek ausgewählten BOP-Operation-Blocks;

2.2 das Hinzufügen zum Operation-Blocks-Schema einer Definition der Beziehungen zwischen den Eingängen und den Ausgängen jedes der ausgewählten BOP-Operation-Blocks;

2.3 die Bestimmung durch den Rechner, auf der Basis der in den Schritten 1.1 bis 2.2 erhaltenen Daten, von Gesamtbilanzen des Verfahrens, wobei diese Gesamtbilanzen einen oder mehrere Parameter enthalten, die aus der Produktion der einen oder mehrerer finaler Substanzen, dem Verbrauch von Rohstoffen oder von Energie ausgewählt sind,

2.4 optional die Wiederholung der Schritte 2.1 und oder 2.2 dann 2.3 bis zum Erhalt verbesserter Gesamtbilanzen;

in Schritt 3,

3.1 die Transformation des Operation-Blocks-Schemas in ein Ausrüstungsschema, das die Industrieanlage repräsentiert, wobei Ausrüstungen aus der BEQGEN-Bibliothek generischer Ausrüstungen oder aus der BEQSPEC-Bibliothek spezifischer Ausrüstungen ausgewählt sind, um die Operationen eines oder mehrerer BOP-Operation-Blocks des Operation-Blocks-Schemas bis zur Transformation aller BOP-Operation-Blocks des Operation-Blocks-Schemas durchzuführen;

3.2 die Definition der Merkmale der verschiedenen generischen oder spezifischen Ausrüstungen des Ausrüstungsschemas, wobei diese Merkmale Produktivitäts-, Volumen-, Reaktions-, Entleerungs- oder Heizzeit- oder Investitions- oder Betriebskostendaten umfassen;

3.3 die Bestimmung durch den Rechner, auf der Basis der in den Schritten 3.1 bis 3.2 erhaltenen Daten, der Betriebsparameter der Industrieanlage;

3.4 optional die Wiederholung der Schritte 3.1 und oder 3.2 dann 3.3 bis zum Erhalt verbesserter Betriebsparameter.

2. Methode nach Anspruch 1, **dadurch gekennzeichnet, dass** man ferner über eine erste MOP1-Operation-Models-Bibliothek verfügt, die die Beziehungen zwischen den Eingängen und den Ausgängen der BOP-Operation-Blocks definiert, und dass in Schritt 2.2 die Definition der Beziehungen zwischen den Eingängen und den Ausgängen von mindestens einem der ausgewählten BOP-Operation-Blocks aus der ersten Bibliothek abgeleitet wird, optional ergänzt durch die Bereitstellung von Parametern dieses Modells durch den Benutzer.

3. Methode nach einem der vorangehenden Ansprüche, **dadurch gekennzeichnet, dass** sie ferner nach Abschluss von Schritt 2.3 oder 2.4 einen Schritt 2.5 umfasst, bei dem die Beziehungen zwischen den Eingängen und den Ausgängen einiger der BOP-Operation-Blocks durch aus einer zweiten MOP2-Operation-Models-Bibliothek abgeleitete Beziehungen ersetzt werden, wobei diese zweite MOP2-Operation-Models-Bibliothek präzisere Merkmale aufweist, die aus den thermodynamischen Informationen oder Phasenzusammensetzungen ausgewählt sind, und die Verfahrensbilanzen gemäß Schritt 2.3 auf der Basis dieser verbesserten Merkmale bestimmt werden.

4. Methode nach einem der vorangehenden Ansprüche, **dadurch gekennzeichnet, dass** man über eine erste MEQ1-Ausrüstungs-Models-Bibliothek verfügt, die Leistungsmerkmale der Ausrüstungen aufweist und dass in Schritt 3.2 die Definition der Merkmale mindestens aus einer der generischen oder spezifischen Ausrüstungen des Ausrüstungsschemas der ersten MEQ1-Ausrüstungs-Models-Bibliothek abgeleitet werden, optional ergänzt durch die Bereitstellung von Parametern dieses Modells durch den Benutzer.

5. Methode nach einem der vorangehenden Ansprüche, **dadurch gekennzeichnet, dass** sie ferner nach Abschluss von Schritt 3.3 oder 3.4 einen Schritt 3.5 umfasst, bei dem die Merkmale einiger der generischen oder spezifischen Ausrüstungen des Ausrüstungsschemas durch aus einer zweiten MEQ2-Ausrüstungs-Models-Bibliothek abgeleitete Merkmale ersetzt werden, wobei die aus dieser zweiten Bibliothek abgeleiteten Models präziser sind und insbesondere erlauben, Umwandlungen in Reaktoren oder Trennleistungen durch Destillation auf der Basis von physikalisch-chemischen Daten zu berechnen, und die Betriebsparameter der Industrieanlage gemäß Schritt 3.3 auf der Basis dieser verbesserten Merkmale bestimmt werden.

6. Methode nach den Ansprüchen 4 oder 5, **dadurch gekennzeichnet, dass** sie ferner nach Abschluss von Schritt 3.3 oder 3.4 einen Schritt 3.5 umfasst, bei dem die Merkmale einiger der verschiedenen generischen oder spezifischen Ausrüstungen des Ausrüstungsschemas durch aus einer dritten MEQ3-Ausrüstungs-Models-Bibliothek abgeleitete Merkmale ersetzt werden, wobei die aus dieser dritten Bibliothek abgeleiteten Models detaillierte Models sind und die Betriebsparameter der Industrieanlage gemäß Schritt 3.3 auf der Basis der verbesserten Merkmale bestimmt werden.

7. Methode nach einem der vorangehenden Ansprüche, **dadurch gekennzeichnet, dass** sie ferner den Bau der geplanten Industrieanlage und/oder die Nutzung des chemischen oder biochemischen Verfahrens in dieser Anlage umfasst.

8. Vorrichtung für die Simulation einer Industrieanlage zur Nutzung eines chemischen oder biochemischen Verfahrens zur Produktion einer oder mehrerer finaler Substanzen bei Einhaltung der Anforderungen, die sich auf Betriebsparameter der Industrieanlage beziehen, umfassend die Produktivität, die Leistung, Qualitätsparameter der Produkte wie Reinheit, Abfallerzeugung, Energieverbrauch, die Investitions- oder Betriebskosten, **dadurch gekennzeichnet, dass** sie Mittel zur Umsetzung einer Methode nach einem der Ansprüche 1 bis 7 umfasst.

9. Vorrichtung nach Anspruch 8, umfassend mindestens einen Rechner, einen Speicher, in dem gespeichert sind

   • eine BBOP-Bibliothek aus BOP-Operation-Blocks, wobei jeder BOP-Operation-Block eine elementare Operation eines chemischen oder biochemischen Verfahrens durch seine Eingänge und seine Ausgänge repräsentiert,
   • eine erste MOP1-Operation-Models-Bibliothek, die die Beziehungen zwischen den Eingängen und den Ausgängen der BOP-Operation-Blocks definiert,
   • eine BEQGEN-Bibliothek generischer Ausrüstungen und eine BEQSPEC-Bibliothek spezifischer Ausrüstungen, wobei die Ausrüstungen imstande sind, Operationen der Operation-Blocks auszuführen,
   • eine erste MEQ1-Ausrüstungs-Models-Bibliothek, die Leistungsmerkmale der Ausrüstungen aufweist,

   Eingangs-/Ausgangsmittel, die geeignet sind, es einem Benutzer zu erlauben, die erforderlichen Daten bereitzustellen und die in einer Methode nach einem der Ansprüche 1 bis 7 bestimmten Gesamtbilanzen und Betriebsparameter zur Kenntnis zu nehmen;
   Rechenmittel, um die in der Methode bestimmten Gesamtbilanzen und Betriebsparameter zu bestimmen.

## Claims

1. A computer-implemented method to simulate an industrial facility for exploiting a chemical or biochemical production method of one or more end substances, paying heed to constraints relating to operating parameters of the industrial facility including productivity, yield, quality parameters of the products such as purity, production of waste, energy consumption, investment or operating costs, wherein there is provided:

   - a library BBOP of blocks of operation BOP, each block of operation BOP representing an elementary operation of a chemical or biochemical method via the inputs and outputs thereof,
   - a library of generic equipment BEQGEN and a library of specific equipment BEQSPEC, said equipment being capable of performing operations of said blocks of operation,

   comprising:

   at step 1:

      1.1 defining by the user of the raw materials entering the method;
      1.2 breakdown of these raw materials into chemical species;
      1.3 providing of information by the user on the chemical species or biological materials produced in the method;

   at step 2:

      2.1 defining a flowsheet of blocks of operation representing the chemical or biochemical method, by arranging blocks of operation BOP selected from the library BBOP of blocks of operation;
      2.2 adding, to the flowsheet of blocks of operation, a definition of the relationships between the inputs and outputs of each of the selected blocks of operation BOP;
      2.3 computer-determining of overall assessments of the method, on a database obtained from steps 1.1 to 2.2, these overall assessments concerning one or more parameters selected from among the production of the end substance(s), the consumption of raw materials or of energy;

2.4 optionally, repeating steps 2.1 and/or 2,2, then 2.3 until improved overall assessments are obtained;

at step 3:

3.1 converting the flowsheet of blocks of operation to a flowsheet of equipment representing said industrial facility, wherein items of equipment are selected from the library of generic equipment BEQGEN or from the library of specific equipment BEQSPEC to perform the operations of one or more blocks of operation BOP in the flowsheet of blocks of operation, up until conversion of all the blocks of operation BOP in the flowsheet of blocks of operation;

3.2 defining the characteristics of the different items of generic or specific equipment in the flowsheet of equipment, these characteristics comprising data on productivity, volume, reaction time, draining or heating, or investment or operating costs;

3.3 computer-determining of said operating parameters of the industrial facility, on the database obtained from steps 3.1 to 3.2

3.4 optionally, repeating steps 3.1 and/or 3.2 then 3,3, up until improved operating parameters are obtained.

2. The method according to claim 1, **characterized in that** there is also provided a first library of models of operation MOP1 defining the relationships between the inputs and outputs of said blocks of operation BOP, and **in that** at step 2.2 the defining of the relationships between the inputs and outputs of at least one of the selected blocks of operation BOP is taken from said first library optionally completed by the providing of parameters of this model by the user.

3. The method according to anyone of the preceding claims **characterized in that**, after step 2.3 or 2.4, it further comprises a step 2.5 wherein the relationships between the inputs and outputs of some blocks of operation BOP are replaced by relationships taken from a second library of models of operation MOP2, this second library of models of operation MOP2 comprising more developed characteristics selected from among thermodynamic or phase composition data, and the overall assessments of the method are determined according to step 2.3. on the basis of these improved characteristics.

4. The method according to anyone of the preceding claims, **characterized in that** there is provided a first library of models of equipment MEQ1 comprising performance characteristics of the equipment, and **in that** at step 3.2 the defining of the characteristics of at least one item of generic or specific equipment in the flowsheet of equipment is taken from said first library of models of equipment MEQ1, optionally completed by the providing of parameters of this model by the user.

5. The method according to anyone of the preceding claims **characterized in that**, after step 3.3 or 3.4, it further comprises a step 3.5 wherein the characteristics of some generic or specific equipment in the equipment flowsheet are replaced by characteristics taken from a second library of models of equipment MEQ2, the models taken from this second library being more developed and in particular allowing calculation of conversions in reactors or performance of distillation separation using physicochemical data, and the operating parameters of the industrial facility are determined according to step 3.3 on the basis of these improved characteristics.

6. The method according to claims 4 or 5 **characterized in that**, after step 3.3 or 3.4, it further comprises a step 3.5 wherein the characteristics of some of the different generic or specific equipment in the equipment flowsheet are replaced by characteristics taken from a third library of models of equipment MEQ3, the models taken from this third library being detailed models, and the operating parameters of the industrial facility are determined according to step 3.3. on the basis of the improved characteristics.

7. The method according to anyone of the preceding claims, **characterized in that** it additionally comprises the implementation of the designed industrial facility and/or exploiting of the chemical or biochemical method in this facility.

8. A device to simulate an industrial facility for exploiting a chemical or biochemical production method of one or more end substances, paying heed to constraints relating to operating parameters of the industrial facility including productivity, yield, quality parameters of products such as purity, production of waste, energy consumption, investment or operating costs, **characterized in that** it comprises means to implement a method according to any of claims 1 to 7.

9. The device according to claim 8 comprising at least one computer, a memory in which there are stored:

• a library BBOP of blocks of operation BOP, each block of operation BOP representing an elementary operation of a chemical or biochemical method via the inputs and outputs thereof;
• a first library of models of operation MOP1 defining the relationships between the inputs and outputs of said blocks of operation BOP;
• a library of generic equipment BEQGEN and a library of specific equipment BEQSPEC, said equipment being capable of performing operations of said blocks of operation;
• a first library of models of equipment MEQ1 comprising performance characteristics of said equipment;

input/output means adapted to enable a user to provide required data and to obtain information on overall assessments and operating parameters determined in a method according to any of claims 1 to 7;
computing means to determine overall assessments and operating parameters determined in said method.

Fig.1

Fig.2

Fig.3

Fig.4

EP 3 566 769 B1

Fig.5

EP 3 566 769 B1

Fig.6

Fig.7

Fig.8

Fig.9

Fig.10

EP 3 566 769 B1

Fig.11

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 8983815 B
- EP 3242226 A
- US 6444854 B
- US 6444854 B1

**Littérature non-brevet citée dans la description**

- **ROGER-MARC NICOUD.** Chromatographic Processes: modeling, simulation and design. Cambridge University Press, 2015